(19) Europäisches Patentamt
European Patent Office
Office européen des brevets

(11) **EP 4 729 514 A1**

(12) **EUROPEAN PATENT APPLICATION**
published in accordance with Art. 153(4) EPC

(43) Date of publication:
**22.04.2026 Bulletin 2026/17**

(21) Application number: **24822731.6**

(22) Date of filing: **13.06.2024**

(51) International Patent Classification (IPC):
***C07D 401/14*** *(2006.01)* ***C07D 471/04*** *(2006.01)*
***C07D 403/14*** *(2006.01)* ***A61K 31/4353*** *(2006.01)*
***A61K 31/495*** *(2006.01)* ***A61K 31/498*** *(2006.01)*
***A61P 35/00*** *(2006.01)*

(52) Cooperative Patent Classification (CPC):
**A61K 31/4353; A61K 31/495; A61K 31/498; A61P 35/00; C07D 401/14; C07D 403/14; C07D 471/04**

(86) International application number:
**PCT/CN2024/098857**

(87) International publication number:
**WO 2024/255782 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**14.06.2023 CN 202310702257**
**08.08.2023 CN 202310990357**
**08.09.2023 CN 202311155643**
**16.11.2023 CN 202311529189**
**01.12.2023 CN 202311633802**
**24.01.2024 CN 202410095352**
**26.03.2024 CN 202410351664**

(71) Applicant: **Haisco Pharmaceutical Group Co., Ltd.**
**Shannan, Tibet 856000 (CN)**

(72) Inventors:
- **LI, Yao**
  **Chengdu, Sichuan 611130 (CN)**
- **CHEN, Lei**
  **Chengdu, Sichuan 611130 (CN)**
- **ZHANG, Haoliang**
  **Chengdu, Sichuan 611130 (CN)**
- **WANG, Jiancheng**
  **Chengdu, Sichuan 611130 (CN)**
- **KANG, Peng**
  **Chengdu, Sichuan 611130 (CN)**
- **FU, Chao**
  **Chengdu, Sichuan 611130 (CN)**
- **ZHAO, Jingzheng**
  **Chengdu, Sichuan 611130 (CN)**
- **TANG, Pingming**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **AWA Denmark A/S**
**Strandgade 56**
**1401 Copenhagen K (DK)**

# (54) BICYCLIC DERIVATIVE PARP INHIBITOR AND USE THEREOF

(57) A compound as represented by formula I-1, a stereoisomer or a pharmaceutically acceptable salt thereof, or a pharmaceutical composition containing same, and the use thereof as a PARP-1 inhibitor in the preparation of a drug for treating related diseases. Each group in formula I-1 is as defined in the description.

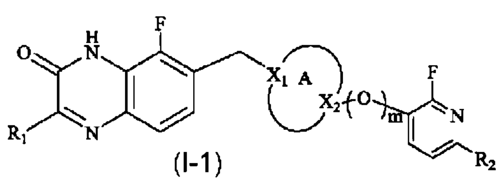

(I-1)

EP 4 729 514 A1

## Description

### Technical Field

The present invention belongs to the field of drugs, and in particular relates to a small molecule compound having a PARP-1 inhibitory activity, or a stereoisomer, pharmaceutically acceptable salt, solvate, co-crystal or deuterated form thereof, and the use thereof in the treatment of related diseases.

### Background Art

Approximately 5% of breast cancer patients are associated with germline mutations in the BRCA1/2 genes (3% in the BRCA1 gene and 2% in the BRCA2 gene). Most breast cancers caused by BRCA1 mutations are triple-negative breast cancers (70%), while BRCA2 mutations are more likely to cause oestrogen receptor-positive breast cancers (70%). The BRCA1/2 genes are tumour suppressor genes and play an important role in DNA damage repair, normal cell growth, etc. Mutations in the genes can inhibit the normal repair ability after DNA damage and cause homologous recombination deficiency (HRD), that is, loss of BRCA function or mutation or loss of function in other homologous recombination-related genes, making repair of DNA double-strand breaks impossible through homologous recombination repair (HRR), eventually leading to cancer.

Poly(ADP-ribose) polymerase (PARP) is a DNA repair enzyme that plays a key role in the DNA repair pathway. PARP is activated by DNA damage and breakage. As a molecular sensor of DNA damage, it has the function of identifying and binding to the DNA break location, thereby activating and catalysing the polyADP ribosylation of the receptor protein and participating in the DNA repair process. PARP plays a key role in the process of DNA single-strand base excision and repair. In HRD tumour cells, DNA double-strand breaks cannot be repaired, and PARP inhibitors block the single-strand repair, resulting in a "synthetic lethal" effect and leading to tumour cell death.

PARP inhibitors have a "trapping" effect on the PARP protein, causing the PARP protein that binds to damaged DNA to be trapped on the DNA, directly causing other DNA repair proteins to be unable to bind, eventually leading to cell death. At present, several PARP inhibitors have been successfully developed, such as olaparib, rucaparib and niraparib. However, adverse reactions limit their ability to be used in combination with chemotherapy drugs. This may be related to the lack of selectivity of marketed PARP inhibitors against the PARP family. The side effects include intestinal toxicity caused by tankyrase inhibition and haematological toxicity caused by PARP-2 inhibition. Therefore, it is of great clinical significance to develop highly selective PARP-1 inhibitors and reduce the toxic and side effects associated with non-selective PARP inhibitors.

### Summary of the Invention

The objective of the present invention is to provide a compound that inhibits PARP-1, or a stereoisomer or pharmaceutically acceptable salt thereof, and the medical application thereof. The compound of the present invention has the advantages of good activity, low toxic and side effects, high safety, strong selectivity, good pharmacokinetics, high bioavailability, etc.

The present invention provides a compound as represented by formula I or formula I-1 below, or a stereoisomer or pharmaceutically acceptable salt thereof,

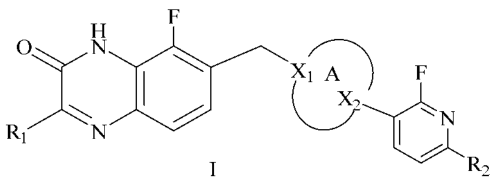

I

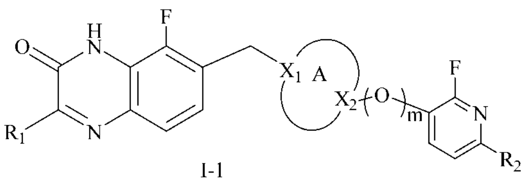

I-1

wherein

ring A is selected from

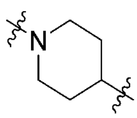,
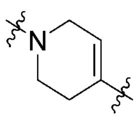,
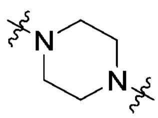 or
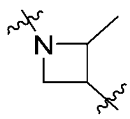;

in some embodiments, ring A is selected from

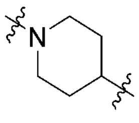, 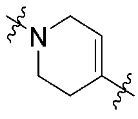 or 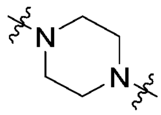;

in some embodiments, ring A is selected from

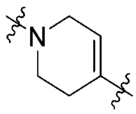 or 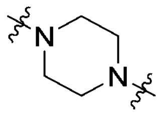;

in some embodiments, ring A is selected from

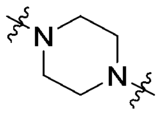 or 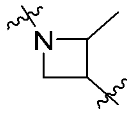;

in some embodiments, ring A is

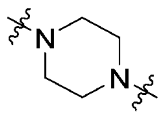;

in some embodiments, ring A is selected from

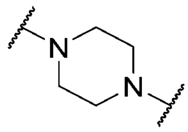 or 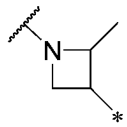;

and in some embodiments, ring A is selected from

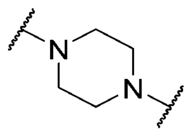 or 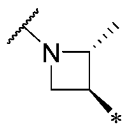,

wherein the "~~~~" end is connected to the left side, and the " * " end is connected to the right side;

$R_1$ is $C_{1-4}$ alkyl or $C_{3-6}$ cycloalkyl, wherein the alkyl or cycloalkyl is optionally substituted with 1-3 groups selected from D, halogen, OH, CN or $NH_2$; in some embodiments, $R_1$ is $C_{1-2}$ alkyl or $C_{3-6}$ cycloalkyl, wherein the alkyl or cycloalkyl is optionally substituted with 1-3 groups selected from D, halogen, OH, CN or $NH_2$; in some embodiments, $R_1$ is $C_{1-2}$ alkyl or $C_{3-4}$ cycloalkyl, wherein the alkyl or cycloalkyl is optionally substituted with 1-3 groups selected from D, F, Cl, OH, CN or $NH_2$; in some embodiments, $R_1$ is methyl, ethyl, isopropyl, cyclopropyl or cyclobutyl, wherein the methyl, ethyl, isopropyl, cyclopropyl or cyclobutyl is optionally substituted with 1, 2 or 3 groups selected from D, F, Cl, OH, CN or $NH_2$; in some embodiments, $R_1$ is methyl, ethyl, cyclopropyl, $-CH_2F$, $-CHF_2$, $-CF_3$,$-CH_2CH_2F$, $-CH_2CHF_2$, $-CH_2CF_3$, $-CHFCH_2F$, $-CHFCHF_2$, $-CHFCF_3$, $-CF_2CH_2F$,$-CF_2CHF_2$, or $-CF_2CF_3$;

in some embodiments, $R_1$ is selected from methyl or ethyl, wherein the methyl or ethyl is optionally substituted with 1, 2 or 3 groups selected from D, F or Cl; and in some embodiments, $R_1$ is selected from $-CH_3$, $-CH_2CH_3$, or $-CHF_2$;

$R_2$ is $-CONHR_A$ or $-NHCOR_A$; in some embodiments, $R_2$ is $-CONHR_A$; and in some embodiments, $R_2$ is $-NHCOR_A$;

each $R_A$ is independently $C_{3-6}$ cycloalkyl, or 5- to 6-membered heteroaryl substituted with 1-3 halogen, wherein the heteroaryl contains 1-3 heteroatoms selected from N, S or O, and the heteroaryl or cycloalkyl is optionally substituted

with 1-3 groups selected from D, =O, $C_{1-4}$ alkyl, $C_{1-4}$ alkoxy, -$NHC_{1-4}$ alkyl,-$CONHC_{1-4}$ alkyl, -$NHCOC_{1-4}$ alkyl, OH, CN, $NH_2$ and $C_{1-4}$ haloalkyl; the cycloalkyl is substituted with at least one =$CH_2$, =$CF_2$, =CHF, =CH($C_{1-6}$ alkyl) or =C($C_{1-6}$ alkyl)$_2$; in some embodiments, each $R_A$ is independently $C_{3-6}$ cycloalkyl, or 5- to 6-membered heteroaryl substituted with 1-3 halogen, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, -$NHC_{1-4}$ alkyl, -$CONHC_{1-4}$ alkyl, -$NHCOC_{1-4}$ alkyl, OH, CN, $NH_2$ and $C_{1-4}$ haloalkyl; the cycloalkyl is substituted with at least one =$CH_2$, =$CF_2$, =CHF, =CH($C_{1-6}$ alkyl) or =C($C_{1-6}$ alkyl)$_2$; in some embodiments, each $R_A$ is independently 5- to 6-membered heteroaryl substituted with 1-3 halogen, or $C_{3-6}$ cycloalkyl, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, -$NHC_{1-4}$ alkyl, -$CONHC_{1-4}$ alkyl, -$NHCOC_{1-4}$ alkyl, OH, CN, $NH_2$ and $C_{1-4}$ haloalkyl; the cycloalkyl is substituted with at least one =$CH_2$ or =$CF_2$; in some embodiments, each $R_A$ is independently 5- to 6-membered heteroaryl substituted with 1-3 halogen, or $C_{3-6}$ cycloalkyl, wherein the heteroaryl contains 1-3 heteroatoms selected from N, S or O, and the heteroaryl is optionally substituted with 1-3 groups selected from D, =O, $C_{1-4}$ alkyl, OH, CN, $NH_2$ or $C_{1-2}$ haloalkyl; the cycloalkyl is substituted with one =$CH_2$, =$CF_2$, =CHF, =CH($C_{1-2}$ alkyl) or =C($C_{1-2}$ alkyl)$_2$; in some embodiments, each $R_A$ is independently 5-membered heteroaryl substituted with 1, 2 or 3 F or Cl,

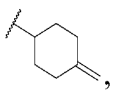, 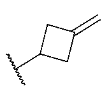, 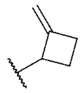,

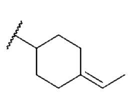, 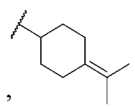, 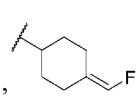, 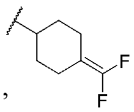,

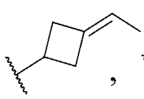, 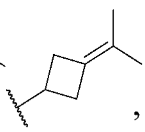, 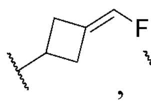, 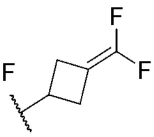, 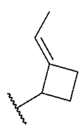, 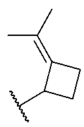, 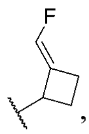, 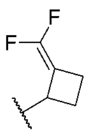,

wherein the heteroaryl contains 1, 2 or 3 heteroatoms selected from N, S or O, and the heteroaryl is optionally substituted with 1, 2 or 3 groups selected from D, =O,-$CH_3$, -$CH_2CH_3$, -$CH_2CH_2CH_3$, -$CH(CH_3)CH_3$, -$CH(CH_3)CH_2CH_3$, OH, CN or $NH_2$; in some embodiments, each $R_A$ is independently 5- to 6-membered heteroaryl substituted with 1-3 halogen,

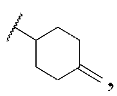, 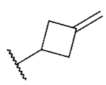 or 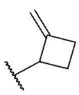,

wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, -$NHC_{1-4}$ alkyl, -$CONHC_{1-4}$ alkyl, -$NHCOC_{1-4}$ alkyl, OH, CN, $NH_2$ and $C_{1-4}$ haloalkyl; in some embodiments, each $R_A$ is independently 5- to 6-membered heteroaryl substituted with 1-3 halogen, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, -$NHC_{1-4}$ alkyl, -$CONHC_{1-4}$ alkyl, -$NHCOC_{1-4}$ alkyl, OH, CN, $NH_2$ and $C_{1-4}$ haloalkyl; in some embodiments, each $R_A$ is independently 5-membered heteroaryl substituted with 1-3 halogen, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, -$NHC_{1-4}$ alkyl, -$CONHC_{1-4}$ alkyl, -$NHCOC_{1-4}$ alkyl, OH, CN, $NH_2$ and $C_{1-4}$ haloalkyl; in some embodiments, each $R_A$ is independently 5-membered heteroaryl substituted with 1, 2 or 3 halogen, wherein the heteroaryl contains 1, 2 or 3 heteroatoms selected from N, S or O, and the heteroaryl is optionally substituted with 1, 2 or 3 groups selected from D, =O, $C_{1-4}$ alkyl, $C_{1-4}$ alkoxy,-$NHC_{1-4}$ alkyl, -$CONHC_{1-4}$ alkyl, -$NHCOC_{1-4}$ alkyl, OH, CN, $NH_2$ and $C_{1-4}$ haloalkyl; in some embodiments, each $R_A$ is independently 5-membered heteroaryl substituted with 1, 2 or 3 F or Cl, wherein the heteroaryl contains 1, 2 or 3 heteroatoms selected from N, S or O, and the heteroaryl is optionally substituted with 1, 2 or 3 groups selected from D, =O, -$CH_3$, -$CH_2CH_3$, -$CH_2CH_2CH_3$,-$CH(CH_3)CH_3$, -$CH(CH_3)CH_2CH_3$, OH, CN or $NH_2$; in some embodiments, each $R_A$ is independently 5-membered heteroaryl substituted with 1, 2 or 3 F, wherein the heteroaryl

contains 1, 2 or 3 heteroatoms selected from N, S or O, and the heteroaryl is optionally substituted with 1, 2 or 3 groups selected from D, =O,-$CH_3$, -$CH_2CH_3$, -$CH_2CH_2CH_3$, -$CH(CH_3)CH_3$, -$CH(CH_3)CH_2CH_3$, OH, CN or $NH_2$; and in some embodiments, each $R_A$ is independently 5-membered heteroaryl substituted with one F, wherein the heteroaryl contains 1, 2 or 3 heteroatoms selected from N, S or O, and the heteroaryl is optionally substituted with 1, 2 or 3 groups selected from D, =O, -$CH_3$, -$CH_2CH_3$, -$CH_2CH_2CH_3$, -$CH(CH_3)CH_3$,-$CH(CH_3)CH_2CH_3$, OH, CN or $NH_2$;
in some embodiments, each $R_A$ is independently selected from

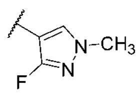,

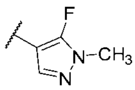, 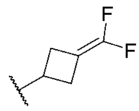, 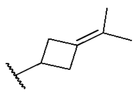 or 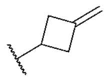;

m is 0 or 1; in some embodiments, m is 0; in some embodiments, m is 1;
the compound is not

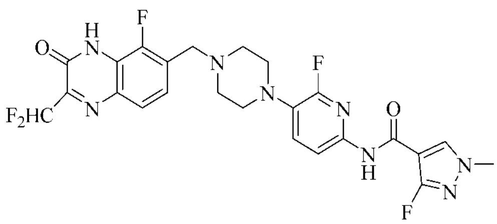.

Specifically, the first technical solution of the present invention provides a compound as represented by formula I, or a stereoisomer or pharmaceutically acceptable salt thereof,

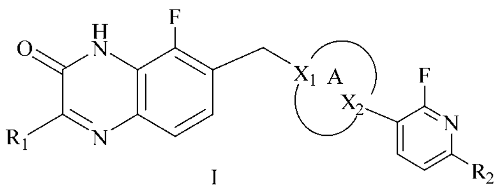

I

wherein

ring A is selected from

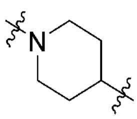, 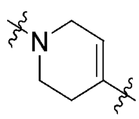 or 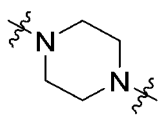;

$R_1$ is $C_{1-4}$ alkyl or $C_{3-6}$ cycloalkyl, wherein the alkyl or cycloalkyl is optionally substituted with 1-3 groups selected from D, halogen, OH, CN or $NH_2$;
$R_2$ is -$CONHR_A$ or -$NHCOR_A$;
each $R_A$ is independently 5- to 6-membered heteroaryl substituted with 1-3 halogen, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, -$NHC_{1-4}$ alkyl, -$CONHC_{1-4}$ alkyl, -$NHCOC_{1-4}$ alkyl, OH, CN, $NH_2$ and $C_{1-4}$ haloalkyl;
the compound is not

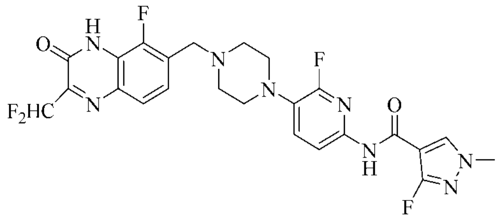

.

The second technical solution of the present invention provides a compound as represented by formula (I), or a stereoisomer or pharmaceutically acceptable salt thereof,
wherein

ring A is

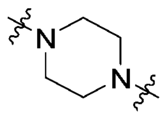

;

$R_1$ is methyl, ethyl, isopropyl, cyclopropyl or cyclobutyl, wherein the methyl, ethyl, isopropyl, cyclopropyl or cyclobutyl is optionally substituted with 1, 2 or 3 groups selected from D, F, Cl, OH, CN or $NH_2$;
each $R_A$ is independently 5-membered heteroaryl substituted with 1, 2 or 3 F or Cl, wherein the heteroaryl contains 1, 2 or 3 heteroatoms selected from N, S or O, and the heteroaryl is optionally substituted with 1, 2 or 3 groups selected from D, =O, $-CH_3$, $-CH_2CH_3$, $-CH_2CH_2CH_3$, $-CH(CH_3)CH_3$, $-CH(CH_3)CH_2CH_3$, OH, CN or $NH_2$.

The third technical solution of the present invention provides a compound as represented by formula I-1, or a stereoisomer or pharmaceutically acceptable salt thereof,

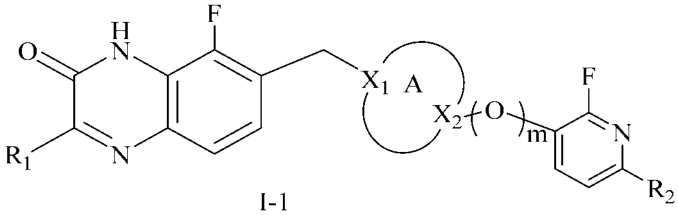

I-1

wherein

ring A is selected from

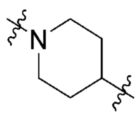
, 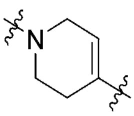
, 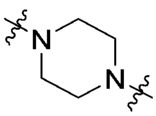
or 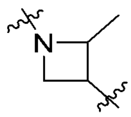
;

$R_1$ is $C_{1-4}$ alkyl or $C_{3-6}$ cycloalkyl, wherein the alkyl or cycloalkyl is optionally substituted with 1-3 groups selected from D, halogen, OH, CN or $NH_2$;
$R_2$ is $-CONHR_A$ or $-NHCOR_A$;
each $R_A$ is independently $C_{3-6}$ cycloalkyl, or 5- to 6-membered heteroaryl substituted with 1-3 halogen, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, $-NHC_{1-4}$ alkyl, $-CONHC_{1-4}$ alkyl, $-NHCOC_{1-4}$ alkyl, OH, CN, $NH_2$ and $C_{1-4}$ haloalkyl; the cycloalkyl is substituted with at least one $=CH_2$, $=CF_2$, =CHF, $=CH(C_{1-6}$ alkyl) or $=C(C_{1-6}$ alkyl$)_2$; in some specific embodiments, each $R_A$ is independently 5- to 6-membered heteroaryl substituted with 1-3 halogen, or $C_{3-6}$ cycloalkyl, wherein the heteroaryl contains 1-3 heteroatoms selected from N, S or O, and the heteroaryl is optionally substituted with 1-3 groups selected from D, =O, $C_{1-4}$ alkyl, $C_{1-4}$ alkoxy, $-NHC_{1-4}$ alkyl, $-CONHC_{1-4}$ alkyl, $-NHCOC_{1-4}$ alkyl, OH, CN, $NH_2$ and $C_{1-4}$ haloalkyl; and the cycloalkyl is substituted with at least one $=CH_2$ or $=CF_2$;
m is 0 or 1;
the compound is not

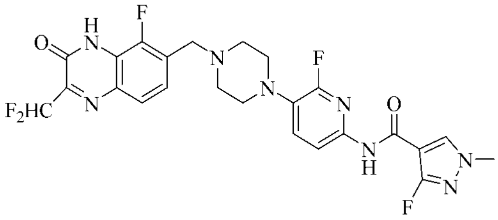.

The fourth technical solution of the present invention provides a compound as represented by formula (I-1), or a stereoisomer or pharmaceutically acceptable salt thereof,

wherein ring A is selected from

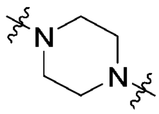 or 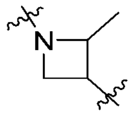;

$R_1$ is $C_{1-2}$ alkyl or $C_{3-4}$ cycloalkyl, wherein the alkyl or cycloalkyl is optionally substituted with 1-3 groups selected from D, F, Cl, OH, CN or $NH_2$; in some specific embodiments, $R_1$ is methyl, ethyl, isopropyl, cyclopropyl or cyclobutyl, wherein the methyl, ethyl, isopropyl, cyclopropyl or cyclobutyl is optionally substituted with 1, 2 or 3 groups selected from D, F, Cl, OH, CN or $NH_2$;

each $R_A$ is independently 5- to 6-membered heteroaryl substituted with 1-3 halogen, or $C_{3-6}$ cycloalkyl, wherein the heteroaryl contains 1-3 heteroatoms selected from N, S or O, and the heteroaryl is optionally substituted with 1-3 groups selected from D, =O, $C_{1-4}$ alkyl, OH, CN, $NH_2$ or $C_{1-2}$ haloalkyl; the cycloalkyl is substituted with one $=CH_2$, $=CF_2$, =CHF, $=CH(C_{1-2}$ alkyl) or $=C(C_{1-2}$ alkyl$)_2$; in some specific embodiments, each $R_A$ is independently 5-membered heteroaryl substituted with 1, 2 or 3 F or Cl,

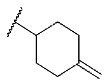, 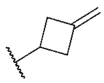, 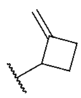,

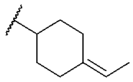, 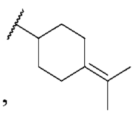, 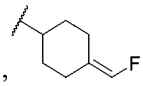, 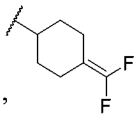,

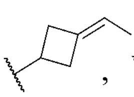, 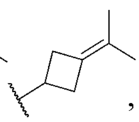, 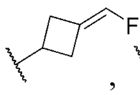, 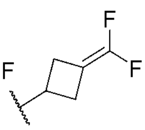, 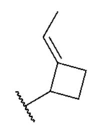, 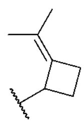, 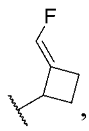, 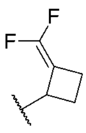,

wherein the heteroaryl contains 1, 2 or 3 heteroatoms selected from N, S or O, and the heteroaryl is optionally substituted with 1, 2 or 3 groups selected from D, =O,$-CH_3$, $-CH_2CH_3$, $-CH_2CH_2CH_3$, $-CH(CH_3)CH_3$, $-CH(CH_3)CH_2CH_3$, OH, CN or $NH_2$; in some specific embodiments, each $R_A$ is independently 5-membered heteroaryl substituted with 1, 2 or 3 F or Cl,

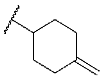, 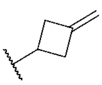 or 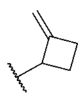,

wherein the heteroaryl contains 1, 2 or 3 heteroatoms selected from N, S or O, and the heteroaryl is optionally substituted with 1, 2 or 3 groups selected from D, =O,$-CH_3$, $-CH_2CH_3$, $-CH_2CH_2CH_3$, $-CH(CH_3)CH_3$, $-CH(CH_3)CH_2CH_3$, OH, CN or $NH_2$.

The fifth technical solution of the present invention provides a compound as represented by formula (I) or formula

(I-1), or a stereoisomer or pharmaceutically acceptable salt thereof,
wherein

ring A is selected from

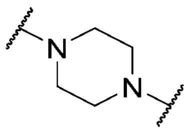 or 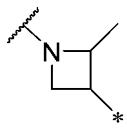,

wherein the "~~~" end is connected to the left side, and the " * " end is connected to the right side;
$R_1$ is selected from $-CH_3$, $-CH_2CH_3$ or $-CHF_2$;
each $R_A$ is independently selected from

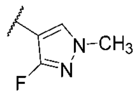, 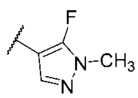, 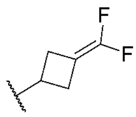,

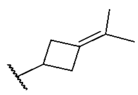 or 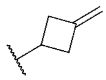.

The sixth technical solution of the present invention provides a compound as represented by formula I, or a stereoisomer or pharmaceutically acceptable salt thereof,

ring A is

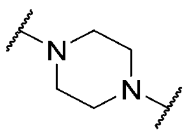,

wherein the "~~~" end is connected to the left side, and the " * " end is connected to the right side;
$R_1$ is selected from $-CH_3$, $-CH_2CH_3$ or $-CHF_2$;
each $R_A$ is independently selected from

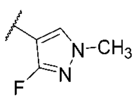, 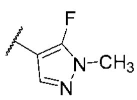, 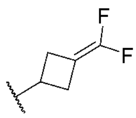,

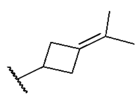 or 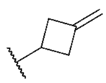.

The seventh technical solution of the present invention provides a compound as represented by formula I-1, or a stereoisomer or pharmaceutically acceptable salt thereof,

ring A is

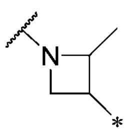,

wherein the "~~~~" end is connected to the left side, and the " * " end is connected to the right side;
$R_1$ is selected from $-CH_3$, $-CH_2CH_3$ or $-CHF_2$;
m is 1;
each $R_A$ is independently selected from

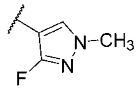, 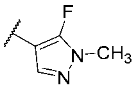, 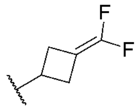,

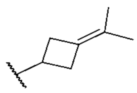 or 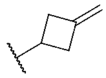.

The compound, or the stereoisomer or pharmaceutically acceptable salt thereof according to the present invention, wherein the compound has a structure selected from one of

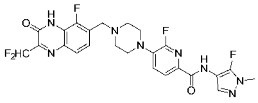 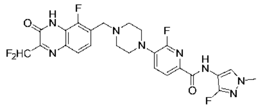 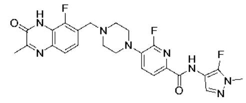 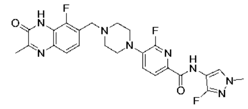

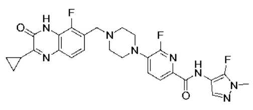 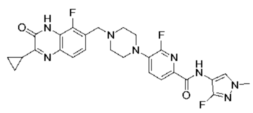 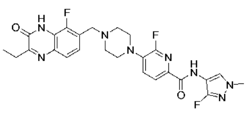 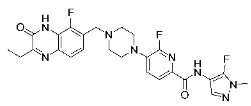

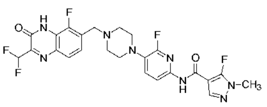 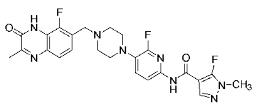 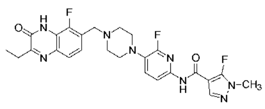 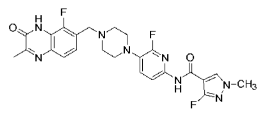

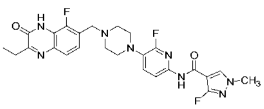 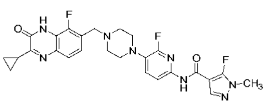 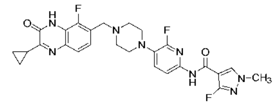

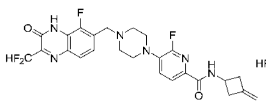 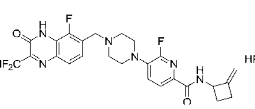 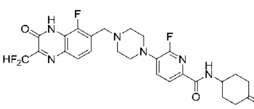 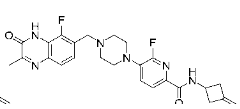

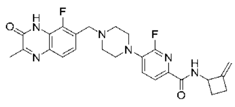 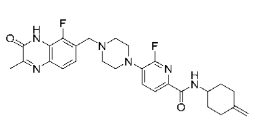 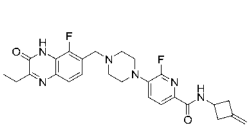 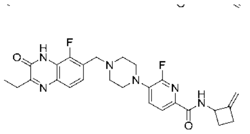

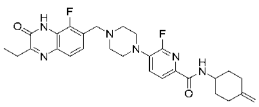 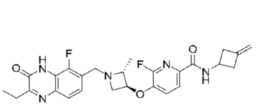 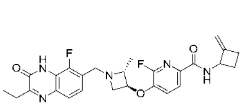 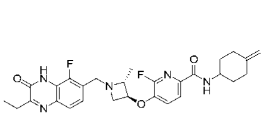

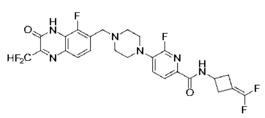 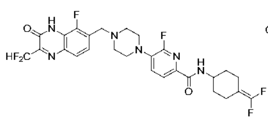 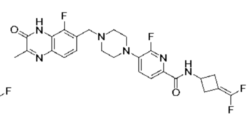 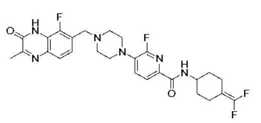

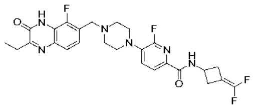
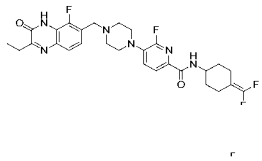
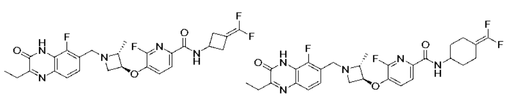

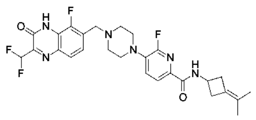
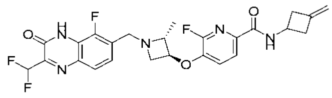
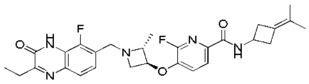

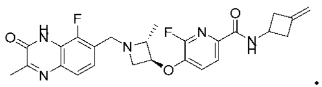.

The present invention further provides a pharmaceutical composition comprising the compound, or the stereoisomer or pharmaceutically acceptable salt thereof according to any of the preceding technical solutions, and a pharmaceutically acceptable carrier and/or excipient. The present invention further provides the use, i.e., the use of the compound, or the stereoisomer or pharmaceutically acceptable salt thereof, or the pharmaceutical composition according to any of the preceding technical solutions in the preparation of a drug for treating/preventing a PARP1-mediated disease.

The PARP1-mediated disease of the present invention is selected from breast cancer, uterine cancer, cervical cancer, ovarian cancer and prostate cancer.

The present invention further provides a method for treating a disease in a mammal, wherein the method comprises administering to a subject a therapeutically effective amount of the compound, or the stereoisomer or pharmaceutically acceptable salt thereof as described above, and a pharmaceutically acceptable excipient and/or carrier, wherein the therapeutically effective amount is preferably 1-1440 mg, and the disease is preferably breast cancer, uterine cancer, cervical cancer, ovarian cancer and prostate cancer.

The present invention further provides a method for treating a disease in a mammal, wherein the method comprises administering to the mammal a therapeutically effective amount of the compound, or the stereoisomer or pharmaceutically acceptable salt thereof, or the pharmaceutical composition according to the present invention. In some embodiments, the mammal as described in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the included compound disclosed in the present application that is required to provide clinically significant reduction of the symptoms of the disease. Examples of the therapeutically effective amount include, but are not limited to 1-1440 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation, wherein the pharmaceutical composition or pharmaceutical preparation comprises a therapeutically effective amount of the compound, or the stereoisomer or pharmaceutically acceptable salt thereof according to the present invention, and an excipient and/or carrier. The pharmaceutical composition can be in a unit dosage form (the amount of the active pharmaceutical ingredient per unit dosage form is also referred to as the "dosage strength"). In some embodiments, the pharmaceutical composition comprises the compound, or the stereoisomer, solvate, deuterated form or pharmaceutically acceptable salt thereof according to the present invention in an amount including but not limited to 1-1440 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg,

230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1440 mg.

The present invention relates to a method for treating a disease in a mammal, wherein the method comprises administering to a subject a therapeutically effective amount of the compound, or the stereoisomer or pharmaceutically acceptable salt thereof according to the present invention, and a pharmaceutically acceptable excipient and/or carrier, wherein the therapeutically effective amount is preferably 1-1440 mg, and the disease is preferably breast cancer, uterine cancer, cervical cancer, ovarian cancer and prostate cancer.

The present invention relates to a method for treating a disease in a mammal, wherein the method comprises administering to a subject a drug, i.e., the compound, or the stereoisomer or pharmaceutically acceptable salt thereof according to the present invention, and a pharmaceutically acceptable excipient and/or carrier in a daily dose of 1-1440 mg/day, wherein the daily dose can be a single dose or divided doses; in some embodiments, the daily dose includes, but is not limited to 10-1440 mg/day, 20-1440 mg/day, 25-1440 mg/day, 50-1440 mg/day, 75-1440 mg/day, 100-1440 mg/day, 200-1440 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1440 mg/day.

The present invention relates to a kit, which may comprise a composition in a single-dose or multi-dose form, wherein the kit comprises the compound, or the stereoisomer or pharmaceutically acceptable salt thereof according to the present invention, and the amount of the compound, or the stereoisomer or pharmaceutically acceptable salt thereof according to the present invention is the same as that in the above-described pharmaceutical composition.

In the present invention, the amount of the compound, or the stereoisomer, or the pharmaceutically acceptable salt thereof according to the present invention is calculated in the form of a free base in each case.

The term "dosage strength" refers to the weight of the active pharmaceutical ingredient per unit dosage form, such as a vial or a tablet.

Synthetic route

Patent document WO 2021013735 A1 introduces a method for preparing a PARP-1 inhibitor, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many standard chemical supply plants (such as those listed above) provide custom synthesis services.

**Terms**

Unless otherwise specified in the present invention, the terms of the present invention have the following meanings.

The carbon, hydrogen, oxygen, sulphur, nitrogen or halogen involved in the groups and compounds of the present invention all include isotopes thereof, and are optionally further replaced by one or more of the corresponding isotopes thereof, wherein the isotopes of carbon include $^{12}C$, $^{13}C$ and $^{14}C$; the isotopes of hydrogen include protium (H), deuterium (D, also known as heavy hydrogen) and tritium (T, also known as superheavy hydrogen); the isotopes of oxygen include $^{16}O$, $^{17}O$ and $^{18}O$; the isotopes of sulphur include $^{32}S$, $^{33}S$, $^{34}S$ and $^{36}S$; the isotopes of nitrogen include $^{14}N$ and $^{15}N$; the isotope of fluorine includes $^{19}F$; the isotopes of chlorine include $^{35}Cl$ and $^{37}Cl$; and the isotopes of bromine include $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number

of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H), which is synonymous with "D".

The term "deuterated" or "deuterated form" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms. "$C_0$" usually indicates the bond.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylidene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to $-CF_3$, $-CH_2Cl$, $-CH_2CF_3$, $-CCl_2$, $CF_3$, etc.

The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic; the bicyclic or polycyclic cycloalkyl may be in the form of a fused ring, a spiro ring, a bridged ring or a combination thereof, and may comprise one or more aromatic rings, but the ring system is non-aromatic as a whole; and the connection site is on a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples include cyclopropyl,

cyclobutyl, cyclopentyl, cyclohexyl, cycloheptyl, butenyl, cyclopentenyl, cyclohexenyl,

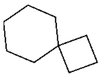, 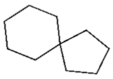, 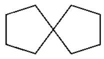, 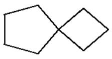, 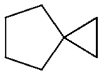, 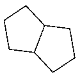, 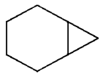,

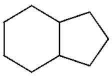, 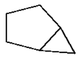, 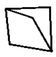, 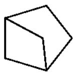, 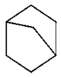, 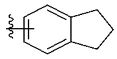,

etc., and the cycloalkyl may be optionally substituted with a substituent.

The term "aryl" refers to an aromatic carbocycle that does not contain heteroatoms, including monocyclic aryl and fused aryl. Generally, the aryl contains 6 to 14 carbon atoms, and further contains 6 to 10 carbon atoms. Non-limiting examples include phenyl, naphthyl, anthryl, and phenanthryl, and the aryl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the connection site. Non-limiting examples include furyl, thienyl, pyrrolyl, oxazolyl, thiazolyl, imidazolyl, pyrazolyl, pyridyl, pyrimidyl, pyridazinyl, pyrazinyl, indolyl, purinyl,

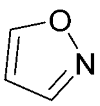, 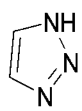, 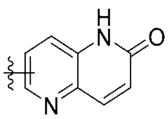, 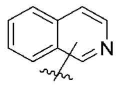, 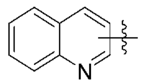,

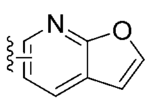, 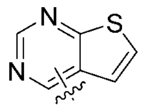, 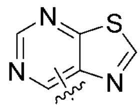, 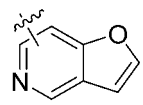,

etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples include oxiranyl, aziridinyl, oxetanyl, azetidinyl, 1,3-dioxolanyl, 1,4-dioxolanyl, 1,3-dioxanyl, piperazinyl, azepanyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo [3.2.1]octanyl, azabicyclo[5.2.0]nonanyl, oxatricyclo[5.3.1.1]dodecyl, azaadamantyl and oxaspiro[3.3]heptanyl,

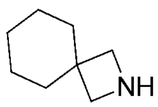, 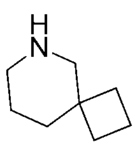, 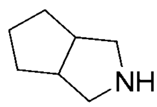, 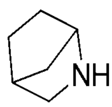, 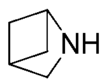,

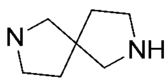, 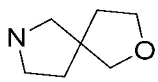, 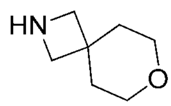, 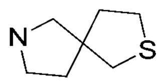,

etc., and the heterocycle may be optionally substituted with a substituent.

Unless otherwise specified, the term "substitution" or "substituent" refers to any substitution at a position allowed by chemical theory, and the number of substituents conforms to the rules of chemical bonding. Exemplary substituents

include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulphonyl, sulphoxide, halo $C_{1-6}$ alkyl, halo $C_{1-6}$ alkoxy, amino, phosphonic acid, $-CO_2(C_{1-6}$ alkyl), -OC(=O)($C_{1-6}$ alkyl), $-OCO_2(C_{1-6}$ alkyl), $-C(=O)NH_2$, -C(=O)N($C_{1-6}$ alkyl$)_2$, -OC(=O)NH($C_{1-6}$ alkyl), -NHC(=O)($C_{1-6}$ alkyl), -N($C_{1-6}$ alkyl)C(=O)($C_{1-6}$ alkyl), $-NHCO_2(C_{1-6}$ alkyl), -NHC(=O)N($C_{1-6}$ alkyl$)_2$, -HC(=O)NH($C_{1-6}$ alkyl), $-NHC(=O)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but used as a diluent, adjuvant, binder and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colourant, a flavouring agent, and a sweetening agent. Examples of pharmaceutically acceptable excipients include, but are not limited to: (1) sugars, such as lactose, glucose and sucrose; (2) starches, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

"Isomer" includes "stereoisomer" and "tautomer". The term "stereoisomer" refers to isomers produced as a result of different spatial arrangement of atoms or atomic groups in molecules that are connected to each other in the same sequence. Stereoisomers include cis-trans isomers, optical isomers and conformational isomers. The term "tautomer" refers to isomers that can be transformed into each other by a reversible chemical reaction called tautomerisation, which is a transformation between functional groups usually caused by the accompanying migration of a hydrogen atom and a $\pi$ bond (a double bond or a triple bond); such as, the following paired compounds: aldehyde/ketone-enol and imine-enamine.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

## Detailed Description of Embodiments

The technical solutions of the present invention will be described in detail below in conjunction with examples, but the protection scope of the present invention includes but is not limited thereto. The structure of the compound is determined by nuclear magnetic resonance (NMR) or (and) mass spectrometry (MS). The NMR shift ($\delta$) is given in the unit

of 10-6 (ppm). NMR is determined with NMR instruments (Bruker Avance III 400 and Bruker Avance 300); the solvents for determination are deuterated dimethyl sulphoxide (DMSO-$d_6$), deuterated chloroform ($CDCl_3$) and deuterated methanol ($CD_3OD$); and the internal standard is tetramethylsilane (TMS).

MS is determined with (Agilent 6120B (ESI) and Agilent 6120B (APCI)); and

HPLC is determined with Agilent 1260DAD high pressure liquid chromatograph (Zorbax SB-C18 100 × 4.6 mm, 3.5 μM).

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm; and for the column chromatography, Yantai Huanghai silica gel of 200-300 mesh silica gel is generally used as a carrier.

Description of abbreviations:

DIPEA: N,N-diisopropylethylamine
HATU: 2-(7-azobenzotriazole)-N,N,N',N'-tetramethyluronium hexafluorophosphate
DMF: N,N-dimethylformamide
DCM: dichloromethane

Example 1

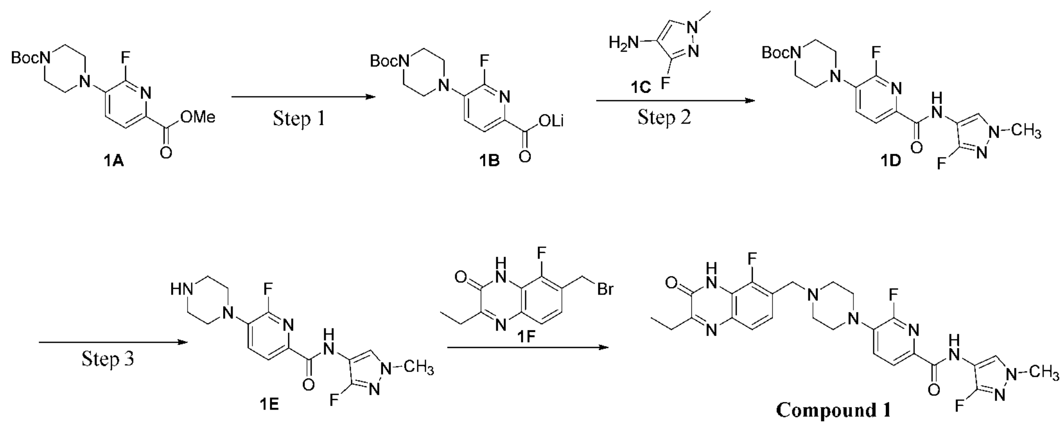


Step 1: Compound 1A (synthesised according to patent US 2022009901 A1) (3.39 g, 10 mmol) was dissolved in tetrahydrofuran (100 mL) and water (10 mL), and lithium hydroxide (400 mg, 10 mmol) was added. The reaction was stirred at room temperature for 2 h. The solvent was removed by distillation under reduced pressure to obtain 1B (3.38 g, 100%) as a white powdery solid, which was directly used in the next step without purification.

LC-MS (ESI): m/z= 324.2 $[M-H]^-$.

Step 2: Compound 1B (324 mg, 1 mmol) and compound 1C (115 mg, 1 mmol; synthesised according to patent WO 2022/212538 A1) were dissolved in DMF (5 mL), and HATU (380 mg, 1 mmol) was added. The mixture was reacted at room temperature for 16 h. The reaction system was poured into water (50 mL), and a large amount of solid products were precipitated. The mixture was suction-filtrated and dried to obtain crude compound 1D (450 mg).

LC-MS (ESI): m/z= 421.4 $[M-H]^-$

Step 3: 450 mg of crude compound 1D was dissolved in a mixed solvent of DCM/TFA = 10/1, and the reaction was stirred at room temperature for 16 h. The reaction system was concentrated to obtain the crude trifluoroacetate of compound **1E** (360 mg).

LC-MS (ESI): m/z = 323.4 $[M+H]^+$

Step 4: The crude trifluoroacetate of compound **1E** (180 mg), and compound **1F** (143 mg, 0.5 mmol; synthesised according to patent WO 2021/260092 Al) were dissolved in acetonitrile (10 mL), and DIPEA (1 mL) was added. The reaction system was warmed to 65°C and reacted for 16 h. The reaction system was concentrated and separated by column chromatography (petroleum ether: ethyl acetate (v : v)=1 : 2) to obtain **compound 1** (77 mg, 29%).

LC-MS (ESI): m/z= 527.2 $[M+H]^+$
$^1$H NMR (400 MHz, DMSO-*d6*) δ 12.42 (s, 1H), 9.82 (s, 1H), 7.93 - 7.88 (m, 1H), 7.86 - 7.82 (m, 1H), 7.63 - 7.53 (m, 2H), 7.33 - 7.27 (m, 1H), 3.71 (s, 5H), 3.25 - 3.17 (m, 4H), 2.86 - 2.78 (m, 2H), 2.65 - 2.57 (m, 4H), 1.25 - 1.19 (m, 3H).

**Example 2**

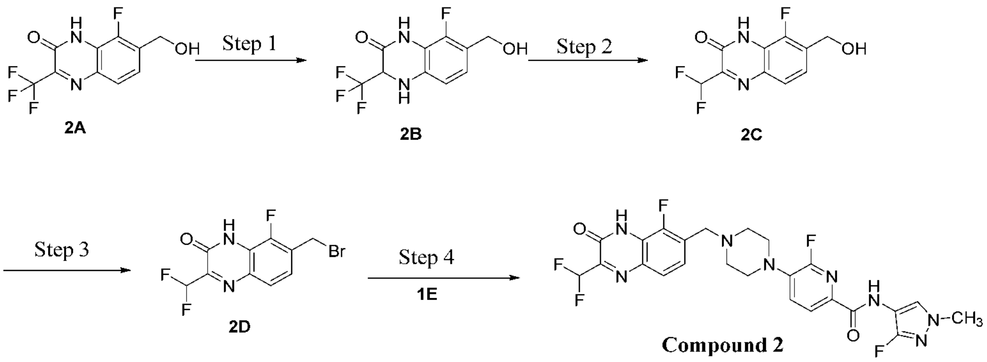


2A 2B 2C 2D Compound 2

Step 1: Compound **2A** (synthesised according to patent US2022009901 A1) (1.3 g, 4.96 mmol) was dissolved in a mixed solution of anhydrous ethanol (26 mL) and water (2 mL). With stirring at room temperature, solid ammonium chloride (3.18 g, 59.47 mmol) and zinc powder (3.24 g, 49.6 mmol) were successively added. After the addition was completed, the mixture was reacted at room temperature for 90 min, and the reaction was monitored by LCMS. After the reaction was completed, a saturated aqueous ammonium chloride solution (30 mL) was added to the reaction solution, and the mixture was filtered through celite. The filter cake was washed with ethyl acetate (100 mL). Ethyl acetate (100 mL) was added to the filtrate, and the aqueous phase was separated. The organic phase was washed with a saturated aqueous sodium chloride solution (150 mL), dried over anhydrous sodium sulphate and filtered to obtain the target compound **2B** (1.30 g, yield: 99.21%).

LCMS m/z = 265.2 $[M+H]^+$
$^1H$ NMR (400 MHz, DMSO-d6) δ 10.96 (s, 1H), 7.16 (s, 1H), 6.88 - 6.84 (m, 1H), 6.63 - 6.61 (m, 1H), 5.01 - 4.98 (m, 1H), 4.87 - 4.80 (m, 1H), 4.39 - 4.40 (m, 2H).

Step 2: Compound **2B** (1.0 g, 3.79 mmol) was dissolved in tetrahydrofuran (20 mL), and an aqueous sodium hydroxide solution (25% (w/w), 5 mL) was added to the reaction solution with stirring at room temperature. After the addition was completed, the mixture was heated at 50°C and reacted for 2 h. The reaction was monitored by TLC (ethyl acetate : petroleum ether (v : v) = 3 : 2). After the reaction was completed, ethyl acetate (40 mL) and water (20 mL) were added to the reaction solution, and the organic phase was separated. The aqueous phase was adjusted to pH 6 with hydrochloric acid (6 mol/L hydrochloric acid). A large number of solids were precipitated and then filtered. The filter cake was dried to obtain the target compound **2C** (0.7 g, yield: 75.64%).

LCMS m/z = 245.2 $[M+H]^+$
$^1H$ NMR (400 MHz, DMSO-*d6*) δ 12.97 (s, 1H), 7.73 - 7.70 (m, 1H), 7.46 - 7.42 (m, 1H), 7.19 - 6.93 (m, 1H), 5.52 - 5.49 (m, 1H), 4.69 - 4.67 (m, 2H).
$^{19}F$ NMR (400 MHz, DMSO-*d6*) δ -122.41(s), -123.46(s).

Step 3: Compound **2C** (488 mg, 2.0 mmol) was dissolved in dichloromethane (20 mL) and cooled to 0°C. Triphenylphosphine (1.57 g, 6.0 mmol) and carbon tetrabromide (1.98 g, 6.0 mmol) were successively added. After the addition was completed, the ice bath was removed, and the mixture was naturally warmed to room temperature and stirred at room temperature for 2 h. The reaction was monitored by LCMS. After the reaction was completed, the reaction solution was directly concentrated and purified by column chromatography (eluent: PE : EA (v : v) = 5 : 1-1 : 1) to obtain the target compound **2D** (530 mg, yield: 86.32%).

LC-MS (ESI): m/z = 307.2, 309.2 $[M+H]^+$.

Step 4: The crude trifluoroacetate of compound **1E** (180 mg), and compound 2D (154 mg, 0.5 mmol) were dissolved in acetonitrile (10 mL), and DIPEA (1 mL) was added. The reaction system was warmed to 65°C and reacted for 16 h. The reaction system was concentrated and separated by column chromatography (petroleum ether : ethyl acetate (v : v) = 1 : 2) to obtain **compound 2** (80 mg, 29%).

LC-MS (ESI): m/z = 549.2 $[M+H]^+$
$^1H$ NMR (400 MHz, DMSO-*d6*) δ 13.00 (s, 1H), 9.82 (s, 1H), 7.94 - 7.88 (m, 1H), 7.86 - 7.81 (m, 1H), 7.75 - 7.69 (m, 1H),

7.63 - 7.56 (m, 1H), 7.46 - 7.39 (m, 1H), 7.22 - 6.91 (m, 1H), 3.76 (s, 2H), 3.72 (s, 3H), 3.25 - 3.17 (m, 4H), 2.65 - 2.59 (m, 4H).

**Example 3**

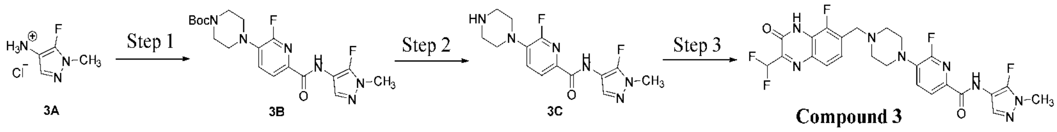


3A 3B 3C Compound 3

Step 1: Compound **1B** (3.01 g, 9.24 mmol) was added to a reaction flask, and dissolved in DMF (30 ml). Then DIPEA (21 ml, 121.6 mmol) and HATU (5.6 g, 14.73 mmol) were added, and the mixture was stirred at room temperature for 20 min; finally compound **3A** (1.4 g, 9.24 mmol; synthesised according to patent WO 2020249664 A1) was added, and the mixture was reacted at room temperature for 1 hour. After the reaction was completed as monitored by TLC, the reaction product was purified by silica gel column chromatography (PE : EA (v : v) = 2 : 1) to obtain compound **3B** (1.1 g, yield: 28.20%).
LC-MS(ESI):m/z = 423.7[M+H]$^+$.
Step 2: Compound **3B** (1.1 g, 2.60 mmol) was added to a reaction flask, and dissolved in DCM (30 ml). Then trifluoroacetic acid (10 ml) was added, and the mixture was reacted at room temperature for 1 hour. After the reaction was completed as monitored by TLC, the reaction product was concentrated under reduced pressure. The residue was washed twice with PE : EA (v : v) = 10 : 1 (100 ml×2) and dried to obtain the crude trifluoroacetate of compound **3C** (800 mg), which was directly used in the next step.
LC-MS(ESI):m/z = 323.3[M+H]$^+$.
Step 3: The trifluoroacetate of compound **3C** (400 mg, 1.24 mmol), **2D** (380 mg, 1.24 mmol) and DIPEA (0.9 ml, 5.0 mmol) were added to a reaction flask, and dissolved in ACN (20 ml). Then the mixture was reacted at 70°C for 2 hours. After the reaction was completed as monitored by TLC, the reaction product was concentrated under reduced pressure, and purified by silica gel column chromatography (DCM : MeOH (v : v) = 10 : 1) to obtain compound 3 (142 mg, yield: 20.88%).
$^1$H NMR (400 MHz, DMSO-*d6*) δ 9.91 (s, 1H), 7.91 (d, 1H), 7.71 (d, 1H), 7.64 - 7.56 (m, 1H), 7.48 - 7.46 (m, 1H), 7.43 - 7.39 (m, 1H), 7.20 - 6.94 (m, 1H), 3.76 (s, 2H), 3.71 (s, 3H), 3.25 - 3.20 (m, 4H), 2.65 - 2.61 (m, 4H).
LC-MS(ESI):m/z = 549.1[M+H]$^+$.

**Example 4**

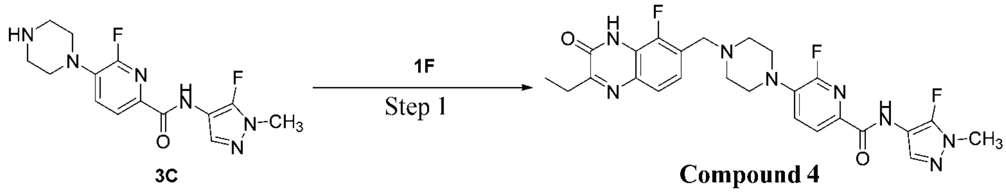


3C Compound 4

Step 1: The trifluoroacetate of compound **3C** (400 mg, 1.24 mmol), **1F** (350 mg, 1.24 mmol) and DIPEA (0.9 ml, 5.0 mmol) were added to a reaction flask, and dissolved in acetonitrile (20 ml). Then the mixture was reacted at 70°C for 2 hours. After the reaction was completed as monitored by TLC, the reaction product was concentrated under reduced pressure, and purified by silica gel column chromatography (DCM : MeOH (v : v) = 10 : 1) to obtain compound 4 (57 mg, yield: 8.73%).
$^1$H NMR (400 MHz, DMSO-d6) δ 9.91 (s, 1H), 7.92 - 7.90 (m, 1H), 7.64 - 7.51 (m, 2H), 7.48 - 7.45 (m, 1H), 7.31 - 7.27 (m, 1H), 3.74 - 3.68 (m, 5H), 3.24 - 3.18 (m, 4H), 2.85 - 2.80 (m, 2H), 2.64 - 2.58 (m, 4H), 1.24 -1.20 (m, 3H).
LC-MS(ESI):m/z = 527.2[M+H]$^+$.

**Example 5**

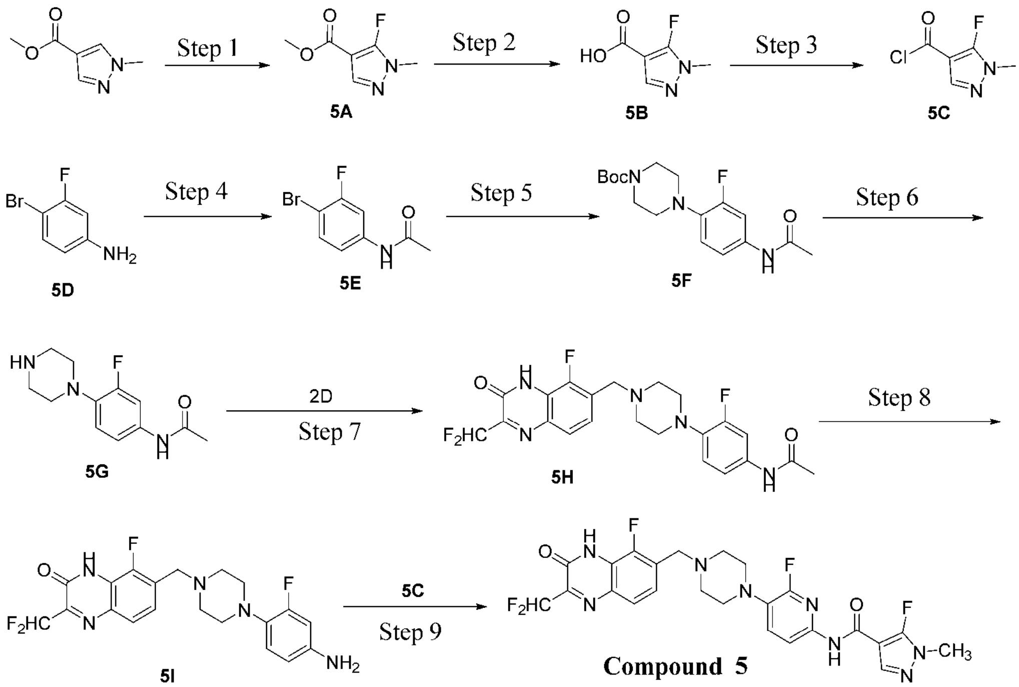

5A 5B 5C
5D 5E 5F
5G 5H
5I Compound 5

Step 1: Methyl 1-methyl-1H-pyrazole-4-carboxylate (5.2 g, 30 mmol) and $AgF_2$ (17.5 g, 120 mmol) were successively added to a 100 mL eggplant-shaped bottle, and then acetonitrile (40 mL) was added. The mixture was reacted at room temperature overnight under $N_2$ atmosphere. The reaction solution was filtered through celite. The filter residue was washed with ethyl acetate, and the filtrate was concentrated. The resulting residue was separated by silica gel column chromatography (petroleum ether : ethyl acetate (v : v) = 10 : 1) to obtain the target compound **5A** (3.9 g, 82.3%).

LC-MS (ESI): m/z = 159.2 $[M+H]^+$.

Step 2: Compound **5A** (1 g, 6.3 mmol) was weighed and dissolved in tetrahydrofuran (20 mL). With stirring, 2 M lithium hydroxide solution (4 mL) was added. The mixture was reacted at room temperature overnight. Upon complete conversion of starting materials as monitored by LCMS, the solution was adjusted to pH 5-6 with 1 M hydrochloric acid. The organic phase was separated, and the aqueous phase was extracted with ethyl acetate (30 mL). The organic phase was combined, dried over anhydrous sodium sulphate, and concentrated. The resulting residue was recrystallised (petroleum ether : ethyl acetate (v : v) = 4 : 1) to obtain the target compound **5B** (624 mg, 68.1%).

LC-MS (ESI): m/z = 143.2 $[M-H]^-$.

Step 3: Compound **5B** (144 mg, 1 mmol) was weighed into a 25 mL eggplant-shaped bottle, and thionyl chloride (5 mL) was added. The mixture was refluxed at 60°C for 2 h, and concentrated to obtain the target compound **5C** (142 mg, crude), which was directly used in the next reaction without further purification.

Step 4: Compound **5D** (4 g, 21 mmol) was dissolved in dichloromethane (50 mL), and pyridine (2.5 mL, 32 mmol) and acetyl chloride (1.7 mL, 23 mmol) were added at room temperature. Upon complete depletion of starting materials as monitored by TLC, the system was concentrated and slurried with ethyl acetate/petroleum ether (v : v) = 1/15 (160 mL) to obtain compound **5E** (4 g, 90%).

LC-MS (ESI): m/z = 233.1 $[M+H]^+$

Step 5: **5E** (4 g, 17 mmol), N-Boc-piperazine (3.8 g, 21 mmol), $Pd_2(dba)_3$(1.6 g, 1.7 mmol), RuPhos (1.4 g, 3.2 mmol) and t-BuOK (4.8 g, 43 mmol) were added to a round bottom flask, and 100 mL of 1,4-dioxane was added. The mixture was purged with nitrogen, heated to reflux, stirred overnight and cooled to room temperature. Silica gel was added. The mixture was concentrated, and the residue was separated by column chromatography (PE : EA (v : v) = 5 : 1 to 1 : 3) to obtain compound **5F** (1.1 g, 19%).

LC-MS (ESI): m/z = 338.1 $[M+H]^+$

Step 6: Compound **5F** (800 mg, 2.4 mmol) was dissolved in TFA/DCM = 1 : 4 (20 mL), and the mixture was reacted at room temperature until complete depletion of starting materials. Then, the system was directly concentrated to obtain the crude trifluoroacetate of compound **5G** (744 mg), which was directly used in the next step.

LC-MS (ESI): m/z = 238.2 $[M+H]^+$

Step 7: The crude compound **5G** (744 mg), and **2D** (675 mg, 2.2 mmol) were dissolved in 30 mL of acetonitrile, and 6 mL of DIPEA was added. The mixture was warmed to 60°C, reacted for 2 h, and cooled to room temperature. The resulting mixture was concentrated to remove most of the acetonitrile. The residue was separated by reverse-phase column chromatography (water/acetonitrile (v : v) = 1 : 1) to obtain compound **5H** (320 mg, 27% over two steps).

LC-MS (ESI): m/z = 464.1 $[M+H]^+$
Step 8: Compound **5H** (320 mg, 0.76 mmol) was dissolved in a mixed solvent of concentrated hydrochloric acid/ethanol = 2 : 1 (12 mL), and the mixture was heated to 70°C, reacted for 1 h, and cooled to room temperature. The system was then directly concentrated to dryness to obtain compound **5I** as a crude hydrochloride (410 mg), which was directly used in the next step.

LC-MS (ESI): m/z = 422.2 $[M+H]^+$
Step 9: The crude **5I** (410 mg) was dispersed in dichloromethane (10 mL), and pyridine (2 mL) was added. With stirring, a solution of **5C** (142 mg, crude) in dichloromethane (5 mL) was dropwise added. The mixture was reacted at room temperature for 2 h. The reaction solution was concentrated. The resulting residue was separated by silica gel column chromatography (dichloromethane : methanol (v : v) = 20 : 1) to obtain compound 5 (147 mg, 35.3% over two steps).

$^1$H NMR (400 MHz, DMSO-$d_6$) δ 12.99 (s, 1H), 10.40 (s, 1H), 8.17 - 8.14 (m, 1H), 7.96 - 7.91 (m, 1H), 7.74 - 7.70 (m, 1H), 7.57 - 7.52 (m, 1H), 7.45 - 7.40 (m, 1H), 7.23 - 6.91 (m, 1H), 3.74 (s, 2H), 3.72 (s, 3H), 3.07 - 3.00 (m, 4H), 2.64 - 2.56 (m, 4H).

LC-MS (ESI): m/z = 549.2 $[M+H]^+$.

**Example 6**

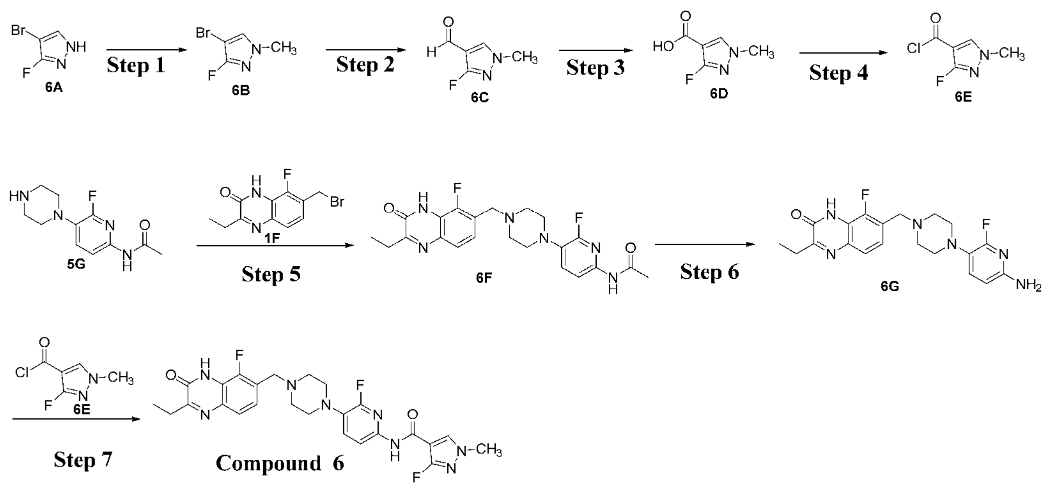


Step 1: Compound **6A** (10 g, 61 mmol) was dissolved in THF (200 mL), and 5 g (60%) of sodium hydride was added at 0°C. The mixture was stirred for 30 min. Iodomethane (6 mL, 96 mmol) was added, and the mixture was slowly warmed to room temperature and reacted for 2 h. The reaction was quenched with a saturated aqueous ammonium chloride solution and extracted with ethyl acetate. The organic phase was collected and concentrated, and the residue was separated by column chromatography (PE : EA = 20 : 1 to 5 : 1) to obtain compound **6B** (6.01 g, 56%).

$^1$H NMR (400 MHz, $CDCl_3$) δ 7.23 - 7.21 (m, 1H), 3.77 - 3.75 (m, 3H).

Step 2: Compound **6B** (3.42 g, 19 mmol) was dissolved in THF (50 mL), and the mixture was cooled to -78°C. n-Butyllithium (11.5 mL, 2.5 M in hexane) was added, and the mixture was stirred at -78°C for 15 min. DMF (3 mL, 39 mmol) was added, and the mixture was reacted at -78°C for 1 h. The system was quenched with ethyl acetate and water, and then extracted with ethyl acetate. The organic phase was collected and concentrated, and the residue was separated by column chromatography (PE : EA = 15 : 1 to 2 : 1) to obtain compound **6C** (840 mg, 35%).

LC-MS (ESI): m/z = 129.1 $[M+H]^+$.

Step 3: Compound **6C** (400 mg, 2.8 mmol) was dissolved in a $KMnO_4$ solution (0.375 M). The mixture was warmed to 75°C, reacted for 1 h and cooled to room temperature. The system was adjusted to a basic pH with an aqueous KOH solution (10%), and solids appeared in the system. The system was filtered, and the filtrate was adjusted to pH = 2 with hydrochloric acid. The system was extracted three times with ethyl acetate/methanol = 10/1 (200 mL). The resulting organic phase was concentrated to obtain crude compound **6D** (300 mg).

LC-MS (ESI): m/z = 145.1 $[M+H]^+$.

Step 4: 20 mL of dichloromethane and 10 mL of thionyl chloride were added to the crude compound **6D**. Under nitrogen atmosphere, the system was warmed to 50°C and reacted for 1 h. The reaction solution was cooled to room temperature and concentrated until no solvent remained, so as to obtain the target compound **6E**, which was directly used in the next reaction.

Step 5: The crude compound **5G** (1.6 g) and **1F** (1.2 g, 4.2 mmol) were dissolved in 50 mL of acetonitrile, and 6 mL

of DIPEA was added. The mixture was warmed to 60°C and reacted for 2 h. The reaction solution was concentrated to remove the solvent, and water (20 mL) was added. The mixture was shaken thoroughly, and then filtered, and the filter cake was dried to obtain the target compound **6F** (768 mg).

LC-MS (ESI): m/z = 443.2 $[M+H]^+$.

Step 6: Compound **6F** (768 mg, 1.73 mmol) was dissolved in a mixed solvent of concentrated hydrochloric acid/ethanol = 2 : 1 (12 mL), and the mixture was heated to 70°C and reacted for 1 h. A saturated sodium bicarbonate solution was dropwise added slowly until no more bubbles were generated, and the mixture was filtered. The filter cake was washed with water, and dried to obtain the target compound **6G** (577 mg, 83.8%).

LC-MS (ESI): m/z = 401.2 $[M+H]^+$.

Step 7: **6G** (577 mg, 1.44 mmol) was dispersed in dichloromethane (20 mL), and pyridine (4 mL) was added. With stirring, a solution of **6E** (142 mg, crude) in dichloromethane (5 mL) was dropwise added. The mixture was reacted at room temperature for 2 h. The reaction solution was concentrated. The resulting residue was separated by silica gel column chromatography (dichloromethane : methanol (v : v) = 20 : 1) to obtain compound **6** (174 mg, 22.9%).

LC-MS (ESI): m/z = 527.2 $[M+H]^+$.

$^1$H NMR (400 MHz, DMSO-$d_6$) δ 11.59 - 10.27 (m, 2H), 7.97 - 7.90 (m, 1H), 7.63 - 7.55 (m, 1H), 7.55 - 7.48 (m, 1H), 7.29 - 7.20 (m, 1H), 6.94 - 6.88 (m, 1H), 3.99 (s, 3H), 3.69 (s, 2H), 3.08 - 3.00 (m, 4H), 2.86 - 2.76 (m, 2H), 2.62 - 2.54 (m, 4H), 1.25 - 1.15 (m, 3H).

**Example 7**

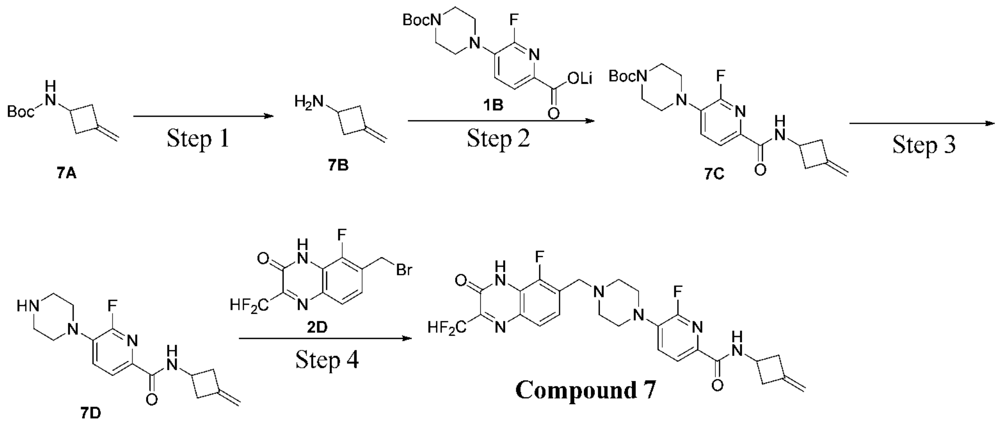


Step 1: Compound **7A** (1 g, 5.46 mmol) was dissolved in a mixed solvent of dichloromethane (20 mL) and trifluoroacetic acid (5 mL), and the reaction was stirred at room temperature for 4 h. The reaction system was concentrated to obtain the crude trifluoroacetate of compound **7B** (800 mg).

LC-MS (ESI): m/z = 84.3 $[M+H]^+$

Step 2: The crude trifluoroacetate of compound **7B** (700 mg, 5.46 mmol), and compound **1B** (1.8 g, 1 mmol) were dissolved in DMF (30 mL), and HATU (2.28 g, 6 mmol) was added. The mixture was reacted at room temperature for 16 h. The reaction solution was poured into water (100 mL), and the mixture was extracted with ethyl acetate (50 mL×3). The combined organic phase was washed with a saturated aqueous sodium chloride solution, dried over anhydrous sodium sulphate, filtered, and concentrated under reduced pressure. The residue was separated and purified by silica gel column chromatography (petroleum ether : ethyl acetate (v/v) = 1 : 1) to obtain compound **7C** (1.5 g, 65.7%).

LC-MS (ESI): m/z = 391.4 $[M+H]^+$

Step 3: Compound **7C** (300 mg, 0.77 mmol) was dissolved in a mixed solvent of dichloromethane (6 mL) and trifluoroacetic acid (1.5 mL), and the reaction was stirred at room temperature for 4 h. The reaction system was concentrated to obtain the crude trifluoroacetate of compound **7D** (250 mg).

LC-MS (ESI): m/z = 291.1 $[M+H]^+$

Step 4: The crude trifluoroacetate of compound **7D** (250 mg), and compound **2D** (212 mg, 0.69 mmol) were dissolved in acetonitrile (10 mL), and DIPEA (298 mg, 2.31 mmol) was added. The reaction system was warmed to 50°C and reacted for 16 h. The reaction system was concentrated and separated by column chromatography (dichloromethane : methanol (v : v) = 20 : 1) to obtain compound **7** (30 mg, 8.4%).

LC-MS (ESI): m/z = 517.2 $[M+H]^+$

¹HNMR (400 MHz, DMSO-*d6*) δ 13.00 (s, 1H), 8.73-8.71 (m, 1H), 7.84-7.82 (m, 1H), 7.73-7.71 (m, 1H), 7.58-7.54 (m, 1H), 7.44-7.40(m, 1H), 7.21-6.94 (m, 1H), 5.07-4.61 (m, 2H), 4.44-4.34 (m, 1H), 3.75 (s, 2H), 3.19-3.17 (m, 4H), 2.99-2.81 (m, 4H), 2.65-2.63 (m, 4H)

**Example 8**

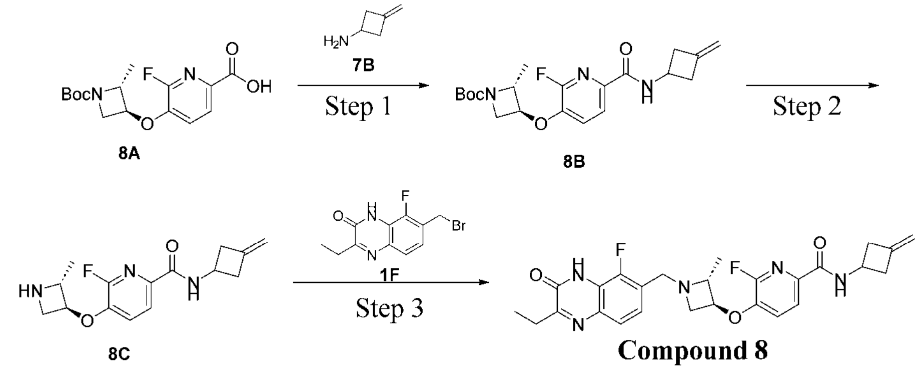

8A 8B 8C Compound 8

Step 1: **8A** (0.5 g, 1.53 mmol, synthesised according to WO 2023056039 A1), **7B** (0.25 g, 3.06 mmol), 2-(7-aza-1H-benzotriazol-1-yl)-1,1,3,3-tetramethyluronium hexafluorophosphate (0.87 g, 2.29 mmol), and diisopropylethylamine (0.59 g, 4.56 mmol) were dissolved in DMF (8 mL), and then the mixture was reacted at room temperature for 2 hours. After the reaction was completed, the reaction solution was subjected to rotary evaporation, and the residue was purified by normal-phase chromatography (petroleum ether : ethyl acetate (v : v) = 4 : 1) to obtain product **8B** (0.3 g, 50%).

LC-MS (ESI): m/z = 336.2[M-56+H]⁺.

Step 2: **8B** (0.25 g, 0.64 mmol) was dissolved in dichloromethane (4 mL), and trifluoroacetic acid (1.53 g, 13.42 mmol) was added. The mixture was reacted at room temperature for 2 hours. After the reaction was completed, the reaction solution was directly subjected to rotary evaporation to obtain crude **8C** (0.18 mg, crude).

LC-MS (ESI): m/z = 292.2[M+H]⁺.

Step 3: Compound **8C** (0.15 g, 0.51 mmol), compound **1F** (0.16 g, 0.56 mmol) and diisopropylethylamine (0.33 g, 2.55 mmol) were dissolved in acetonitrile (8 mL), and then the mixture was reacted at 55°C for 3 hours. After the reaction was completed, the reaction solution was concentrated to dryness and directly subjected to rotary evaporation and preparative HPLC ( instrument: waters 2767 preparative liquid chromatographic instrument; chromatographic column: SunFire @ Prep C18 (19 mm × 150 mm); composition of mobile phase: mobile phase A: acetonitrile, and mobile phase B: water (containing 5% ammonium acetate); gradient: a gradient of 10%-50% acetonitrile) to obtain compound **8** (50 mg, 19%).

LC-MS (ESI): m/z = 496.2 [M+H]⁺.
¹H NMR (400 MHz, DMSO-$d_6$) δ 8.80 (d, 1H), 7.85 (d, 1H), 7.64 - 7.57 (m, 1H), 7.53 (d, 1H), 7.30 - 7.23 (m, 1H), 4.85 - 4.77 (m, 2H), 4.64 - 4.55 (m, 1H), 4.44 - 4.33 (m, 1H), 3.91 (d, 1H), 3.84 - 3.78 (m, 1H), 3.70 (d, 1H), 3.44 - 3.34 (m, 1H), 2.97 - 2.86 (m, 4H), 2.85 - 2.77 (m, 3H), 1.24 - 1.19 (m, 6H).

**Example 9**

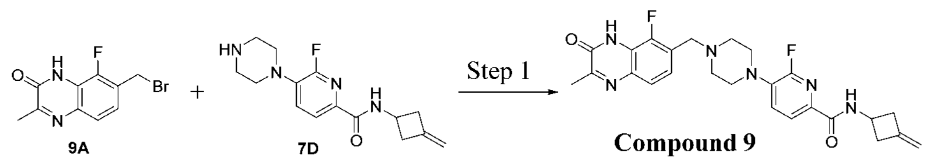

9A 7D Compound 9

Step 1: Compound **9A** (200 mg, 0.74 mmol, synthesised according to patent US 20220009901 A1), **7D** (215 mg, 0.74 mmol) and DIPEA (0.4 ml, 2.22 mmol) were added to a reaction flask, and then dissolved in acetonitrile (5 ml). The mixture was reacted at 50°C for 2 hours. After the reaction was completed as monitored by TLC, the reaction solution was cooled to room temperature, and filtered. The filter cake was washed once with acetonitrile (5 ml), and then washed once

with ethyl acetate (10 ml). The filter cake was collected and dried to obtain compound 9 (140 mg, yield 39.44%).

LC-MS(ESI):m/z = 481.3[M+H]$^+$.

$^1$H NMR (400 MHz, DMSO) δ 12.42 (s, 1H), 8.74 - 8.67 (m, 1H), 7.85 - 7.80 (m, 1H), 7.58 - 7.49 (m, 2H), 7.32 - 7.25 (m, 1H), 4.83 - 4.78 (m, 2H), 4.44 - 4.33 (m, 1H), 3.70 (s, 2H), 3.20 - 3.13 (m, 4H), 2.94 - 2.84 (m, 4H), 2.62 - 2.55 (m, 4H), 2.41 (s, 3H).

**Example 10**

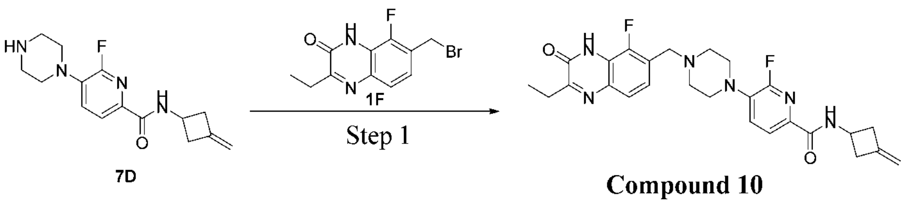


7D

**Compound 10**

Step 1: The crude trifluoroacetate of compound 7D (300 mg), and compound 1F (250 mg, 0.88 mmol) were dissolved in acetonitrile (10 mL), and DIPEA (1 mL) was added. The reaction system was warmed to 50°C and reacted for 2 h. The reaction system was concentrated and separated by column chromatography (DCM : MeOH (v : v) = 15 : 1) to obtain compound 10 (105 mg, 24.14%).

LC-MS (ESI): m/z = 495.4 [M+H]$^+$

$^1$H NMR (400 MHz, DMSO-$d_6$) δ 12.43 (s, 1H), 8.73 - 8.71 (m, 1H), 8.03 - 7.77 (m, 1H), 7.70 - 7.45 (m, 2H), 7.31 - 7.27 (m, 1H), 5.14 - 4.75 (m, 2H), 4.42 - 4.36 (m, 1H), 3.71 (s, 2H), 3.16 - 3.18 (m, 4H), 2.88 - 2.94 (m, 4H), 2.824 - 2.79(m, 2H), 2.60 - 2.58 (m, 4H), 1.24 - 1.20(m, 3H).

Example 11

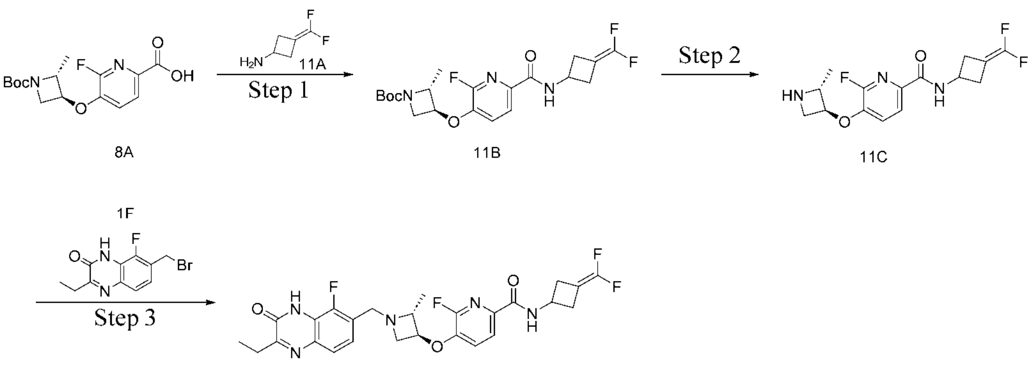


8A

11B

11C

Compound 11

Step 1: 8A (0.15 g, 0.46 mmol; synthesised according to WO 2023056039 A1), 11A (0.077 g, 0.64 mmol; synthesised according to WO 2023025324 A1), 2-(7-aza-1H-benzotriazol-1-yl)-1,1,3,3-tetramethyluronium hexafluorophosphate (0.26 g,0.69 mmol), and diisopropylethylamine (0.18 g,1.38 mmol) were dissolved in N,N-dimethylformamide (8 mL), and the mixture was reacted at room temperature for 2 hours. After the reaction was completed, the reaction solution was diluted in water, and extracted three times with ethyl acetate. The organic phases were combined and subjected to rotary evaporation, and the residue was purified by normal-phase chromatography (petroleum ether : ethyl acetate = 4 : 1) to obtain product **11B** (0.18 g, 91.6%).

LC-MS (ESI): m/z = 372.0[M-56+H]$^+$.

Step 2: **11B** (0.040 g, 0.094 mmol) was dissolved in dichloromethane (2 mL), and trifluoroacetic acid (0.77 g, 6.71 mmol) was added. The mixture was reacted at room temperature for 1 hour. After the reaction was completed, the reaction solution was directly subjected to rotary evaporation to obtain crude **11C** (0.027 g, crude).

LC-MS (ESI): m/z = 328.2[M+H]$^+$.

Step 3: **11C** (0.027 g, 0.082 mmol), **1F** (0.028 g, 0.098 mmol) and diisopropylethylamine (0.042 g, 0.33 mmol)

were dissolved in acetonitrile (5 mL), and then the mixture was reacted at 50°C for 3 hours. After the reaction was completed, the reaction solution was concentrated to dryness, and the residue was purified by preparative HPLC ( instrument: waters 2767 preparative liquid chromatographic instrument; chromatographic column: SunFire @ Prep C18 (19 mm × 150 mm); composition of mobile phase: mobile phase A: acetonitrile, and mobile phase B: water (containing 5% ammonium acetate); gradient: a gradient of 10%-50% acetonitrile) to obtain compound 11 (11 mg, 25%).

LC-MS (ESI): m/z = 532.7 $[M+H]^+$.

$^1H$ NMR (400 MHz, DMSO-$d_6$) δ 8.96 (d, 1H), 7.86 (d, 1H), 7.66 - 7.58 (m, 1H), 7.53 (d, 1H), 7.31 - 7.23 (m, 1H), 4.64 - 4.47 (m, 2H), 3.91 (d, 1H), 3.85 - 3.79 (m, 1H), 3.70 (d, 1H), 3.44 - 3.36(m, 1H), 3.00 - 2.88 (m, 4H), 2.85 - 2.78 (m, 3H), 1.23 (d, 3H), 1.21 - 1.19 (m, 3H).

**Example 12**

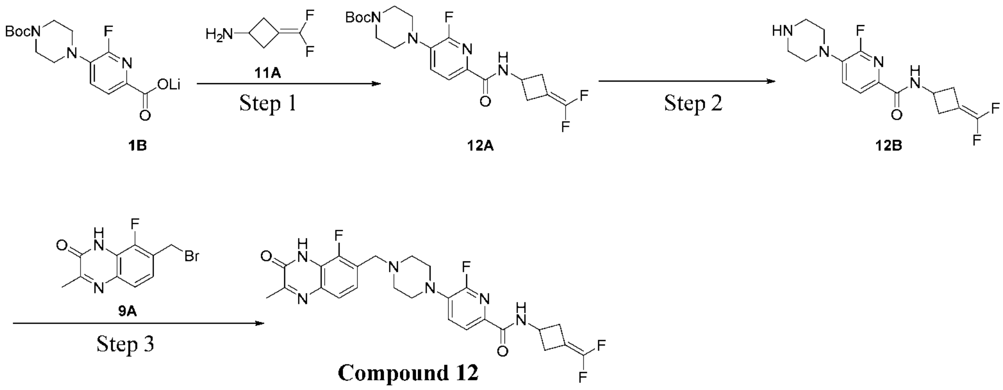


1B 12A 12B

Compound 12

Step 1: Compound **1B** (950 mg, 2.86 mmol) was dissolved in DMF (10 mL), and then HATU (1.53 g, 4.03 mmol), diisopropylethylamine (2 mL) and compound 11A (400 mg, 3.36 mmol) were successively added. After the addition was completed, the mixture was reacted at room temperature for 16 h. The reaction solution was poured into water (100 mL), and the mixture was extracted with ethyl acetate (50 mL×3). The combined organic phase was washed with a saturated aqueous sodium chloride solution, dried over anhydrous sodium sulphate, filtered, and concentrated under reduced pressure to obtain a crude. The crude was slurried with a mixed solvent of ethyl acetate and petroleum ether (EA : petroleum ether = 1 : 2), filtered, and dried to obtain the target compound **12A** (826 mg, 56.98%). LC-MS (ESI): m/z = 427.2 $[M+H]^+$.

Step 2: Compound **12A** (213 mg, 0.5 mmol) was weighed and dissolved in dichloromethane (5 mL), and then trifluoroacetic acid (1 mL) was added. After the addition was completed, the mixture was stirred for 2 h at room temperature, and concentrated under reduced pressure to remove dichloromethane and excess trifluoroacetic acid, so as to obtain the trifluoroacetate of compound **12B** (160 mg), wherein the crude was directly used in the next reaction.

LC-MS (ESI): m/z = 327.1$[M+H]^+$.

Step 3: The trifluoroacetate of compound **12B** (160 mg) was weighed and dissolved in acetonitrile (20 mL), and then diisopropylethylamine (2 mL) and compound **9A** (140 mg, 0.5 mmol) were added. After the addition was completed, the mixture was heated to 65°C and reacted for 3 h. After the reaction was completed, the reaction solution was directly subjected to hot filtration. The filter cake was washed with acetonitrile, and then dried to obtain the target compound **12** (160 mg, 62.01%).

LC-MS (ESI): m/z = 517.2$[M+H]^+$.

$^1H$ NMR (400 MHz, DMSO-$d_6$) δ 12.43 (s, 1H), 8.99 - 8.97 (m, 1H), 7.85 - 7.83 (m, 1H), 7.62 - 7.44 (m, 2H), 7.39 - 7.15 (m, 1H), 4.56 - 4.50 (m, 1H), 3.70 (s, 2H), 3.18 - 3.16 (m, 4H), 3.01 - 2.81 (m, 4H), 2.60 - 2.61 (m, 4H), 2.42 (s, 3H).

**Example 13**

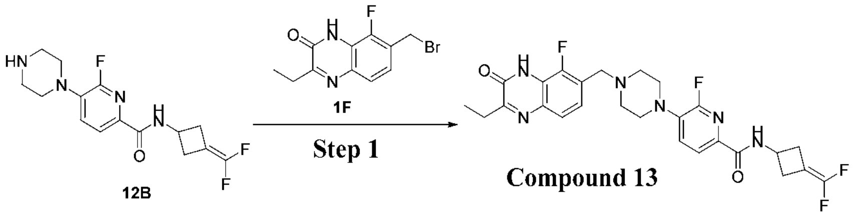


12B

Compound 13

Step 1: The crude trifluoroacetate of compound **12B** (250 mg), and compound **1F** (142 mg, 0.5 mmol) were dissolved in acetonitrile (10 mL), and DIPEA (322 mg, 2.5 mmol) was added. The reaction system was warmed to 65°C and reacted for 2 h. The reaction system was concentrated and separated by column chromatography (dichloromethane : methanol (v : v) = 20 : 1) to obtain compound **13** (140 mg, 52.8%).

LC-MS (ESI): m/z = 531.3 $[M+H]^+$
$^1$H NMR (400 MHz, DMSO-d6) δ 12.43 (s, 1H), 8.90 - 8.88 (m, 1H), 7.84 - 7.82 (m, 1H), 7.57 - 7.53 (m, 2H), 7.31 - 7.27 (m, 1H), 4.56 - 4.50 (m, 1H), 3.70 (s, 2H), 3.18 (s, 4H), 2.95 - 2.90 (m, 4H), 2.84 - 2.79 (m, 2H), 2.59 (s, 4H), 1.23 - 1.20 (m, 3H)

**Example 15**

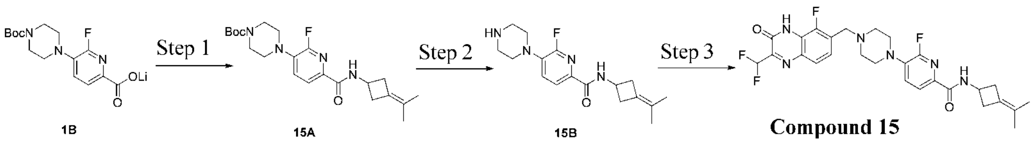


1B 15A 15B Compound 15

Step 1: Compound **1B** (170 mg, 0.51 mmol), 3-(difluoromethylene)cyclobutanaminium trifluoroacetate (180 mg, 0.76 mmol, synthesised according to patent WO 2023025324 A1) and DIPEA (0.9 ml, 5.10 mmol) were added to a reaction flask and dissolved in DMF (10 ml), and then HATU (230 mg, 0.61 mmol) was added. The mixture was reacted at room temperature for 2 hours. After the reaction was completed as monitored by TLC, the reaction solution was diluted in water (50 ml), and extracted twice with ethyl acetate (40 ml×2). The organic phases were combined and dried, and concentrated under reduced pressure. The resulting residue was purified by silica gel column chromatography (PE : EA = 1 : 1) to obtain compound **15A** (180 mg, yield: 84.51%).

LC-MS(ESI):m/z = 419.1$[M+H]^+$.

Step 2: Compound **15A** (180 mg, 0.43 mmol) was added to a reaction flask and dissolved in DCM (10 ml), and then trifluoroacetic acid (1 ml) was added. The mixture was reacted at room temperature for 3 hours. After the reaction was completed as monitored by TLC, the reaction solution was concentrated under reduced pressure to obtain compound **15B** (200 mg, trifluoroacetate, directly used in the next step).

LC-MS(ESI):m/z = 319.1$[M+H]^+$.

Step 3: Compound **15B** (200 mg, 0.46 mmol) and compound **2D** (140 mg, 0.46 mmol) were added to a reaction flask and dissolved in acetonitrile (10 ml), and then DIPEA (0.4 ml, 2.30 mmol) was added. The mixture was reacted at 80°C for 3 hours. After the reaction was completed as monitored by TLC, the reaction solution was cooled to room temperature, and concentrated under reduced pressure. The resulting residue was purified by C18 column (A/B = 0.1% $AcONH_4$ in H2O/ACN, B: 10% to 60%) to obtain compound **15** (52 mg, yield: 20.80%).

1H NMR (400 MHz, DMSO-d6) δ 8.65 - 8.55 (m, 1H), 7.87 - 7.78 (m, 1H), 7.74 - 7.65 (m, 1H), 7.61 - 7.50 (m, 1H), 7.44 - 7.34 (m, 1H), 7.20 - 6.90 (m, 1H), 4.36 - 4.25 (m, 1H), 3.74 (s, 2H), 3.19 - 3.15 (m, 4H), 2.92 - 2.82 (m, 2H), 2.75 - 2.66 (m, 2H), 2.64 - 2.57(m, 4H), 1.50 (s, 6H).

LC-MS(ESI):m/z = 545.4$[M+H]^+$.

**Example 16**

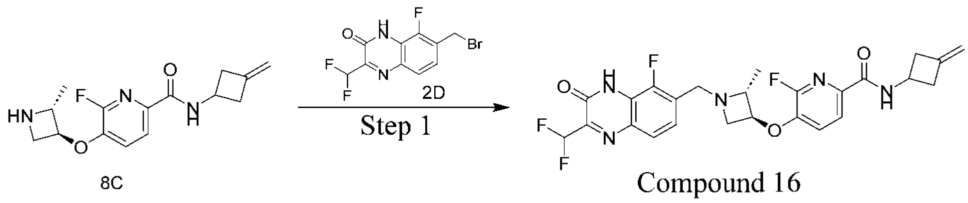


8C

Compound 16

Step 1: **8C** (0.15 g, 0.51 mmol), **2D** (0.17 g, 0.56 mmol) and N,N-diisopropylethylamine (0.2 g, 1.53 mmol) were dissolved in acetonitrile (8 mL), and then the mixture was reacted at 55°C for 3 hours. After the reaction was completed, the reaction solution was directly subjected to rotary evaporation and purified by HPLC ( instrument: waters 2767 preparative liquid chromatographic instrument; chromatographic column: SunFire @ Prep C18 (19 mm × 150 mm); composition of mobile phase: mobile phase A: acetonitrile, and mobile phase B: water (containing 5% ammonium acetate); gradient: a gradient of 10%-50% acetonitrile) to obtain compound **16** (70 mg, 26%).

LC-MS (ESI): m/z = 518.3 $[M+H]^+$.

$^1H$ NMR (400 MHz, Chloroform-d) δ 10.00 (s, 1H), 8.00 (d, 1H), 7.78 (d, 1H), 7.69 (d, 1H), 7.43 (s, 1H), 7.23 - 7.16 (m, 1H), 7.07 - 6.74 (m, 1H), 4.92-4.86 (m, 2H), 4.60 - 4.41 (m, 2H), 4.11 - 3.78 (m, 3H), 3.55 (s, 1H), 3.23 - 3.08 (m, 2H), 2.99 (s, 1H), 2.81-2.66 (m, 2H), 1.29 (d, 3H).

## Example 17

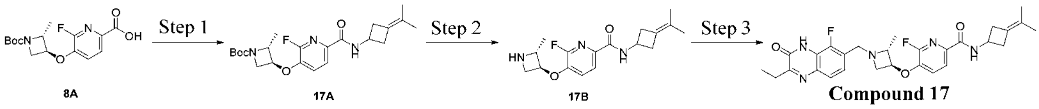


8A 17A 17B **Compound 17**

Step 1: Compound **8A** (150 mg, 0.46 mmol), 3-(difluoromethylene)cyclobutanaminium trifluoroacetate (160 mg, 0.69 mmol, synthesised according to patent WO 2023025324 A1) and DIPEA (0.8 ml, 4.60 mmol) were added to a reaction flask and dissolved in DMF (10 ml), and then HATU (210 mg, 0.55 mmol) was added. The mixture was reacted at room temperature for 2 hours. After the reaction was completed as monitored by TLC, the reaction solution was diluted in water (50 ml), and extracted twice with ethyl acetate (40 ml×2). The organic phases were combined and dried, and concentrated under reduced pressure. The resulting residue was purified by silica gel column chromatography (PE : EA = 1 : 1) to obtain compound **17A** (80 mg).

LC-MS(ESI):m/z = 364.1$[M-56+H]^+$.

Step 2: Compound **17A** (80 mg, 0.19 mmol) was added to a reaction flask and dissolved in DCM (5 ml), and then trifluoroacetic acid (0.5 ml) was added. The mixture was reacted at room temperature for 3 hours. After the reaction was completed as monitored by TLC, the reaction solution was concentrated under reduced pressure to obtain compound **15B** (100 mg, trifluoroacetate, directly used in the next step).

LC-MS(ESI):m/z = 320.3$[M+H]^+$.

Step 3: Compound **17B** (100 mg, 0.23 mmol) and compound **1F** (65 mg, 0.23 mmol) were added to a reaction flask and dissolved in acetonitrile (10 ml), and then DIPEA (0.2 ml, 1.15 mmol) was added. The mixture was reacted at 80°C for 3 hours. After the reaction was completed as monitored by TLC, the reaction solution was cooled to room temperature, and concentrated under reduced pressure. The resulting residue was purified by C18 column (A/B = 0.1% $AcONH_4$ in H2O/ACN, B: 10% to 60%) to obtain compound **17** (5 mg, yield: 4.17%).

$^1H$ NMR (400 MHz, DMSO-d6) δ 12.40 (s, 1H), 8.72 - 8.67 (m, 1H), 7.86 - 7.82 (m, 1H), 7.64 - 7.50 (m, 2H), 7.29 - 7.23 (m, 1H), 4.63 - 4.56 (m, 1H), 4.34 - 4.27 (m, 1H), 3.94 - 3.88 (m, 1H), 3.84 - 3.79 (m, 1H), 3.73 - 3.67 (m, 1H), 3.42 - 3.37 (m, 1H), 2.90 - 2.78 (m, 5H), 2.76 - 2.69 (m, 2H), 1.50 (s, 6H), 1.23 - 1.17 (m, 6H).

LC-MS(ESI):m/z = 524.3$[M+H]^+$.

## Example 18

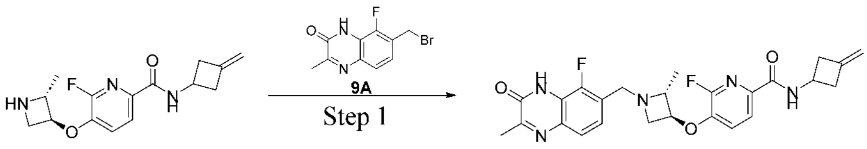

8C
Compound 18

Step 1: Compound **8C** (0.15 g, 0.51 mmol), compound **9A** (0.14 g, 0.51 mmol) and diisopropylethylamine (0.26 g, 2.04 mmol) were dissolved in acetonitrile (5 mL), and then the mixture was reacted at 70°C for 16 hours. After the reaction was completed, the reaction solution was concentrated to dryness and directly subjected to rotary evaporation and preparative HPLC ( instrument: waters 2767 preparative liquid chromatographic instrument; chromatographic column: SunFire @ Prep C18 (19 mm × 150 mm); composition of mobile phase: mobile phase A: acetonitrile, and mobile phase B: water (containing 5% ammonium acetate); gradient: a gradient of 10%-50% acetonitrile) to obtain compound **18** (0.12 g, yield: 48.40%).

LC-MS (ESI): m/z = 482.4$[M+H]^+$.

$^1H$ NMR (400 MHz, DMSO-d6) δ 8.80 (d, 1H), 7.85 (d, 1H), 7.65 - 7.57 (m, 1H), 7.49 (d, 1H), 7.29 - 7.22 (m, 1H), 4.84 - 4.77 (m, 2H), 4.62 - 4.57 (m, 1H), 4.41 - 4.36 (m, 1H), 3.91 (d, 1H), 3.83 - 3.80 (m, 1H), 3.70 (d, 1H), 3.43 - 3.36 (m, 1H), 2.92 - 2.81(m, 5H), 2.41 (s, 3H), 1.20 (d, 3H).

**Biological test**

**1. PARP1 enzyme activity assay**

The inhibition of compounds on PARP1/2 enzyme activity was tested using the FP method. The substrate tracer fluorescent probe (from ICE BIOSCIENCE INC.) in the reaction was prepared in a reaction buffer (50 mM Tris (pH 8.0), 10 mM MgCl2, 150 mM NaCl, 0.001% Triox-100). PARP1/2 (BPS, Cat #80501/80502) was added to the reaction buffer, and gently mixed. The final concentrations of PARP1/2 and the fluorescent probe in the reaction mixture were 5/10 nM and 2.5 nM, respectively. Positive reference Olaparib was serially diluted 3-fold, from an initial concentration of 1 μM, to generate 10 concentration points. 0.1 μL of the compound in 100% DMSO was delivered to a 384-well plate (Corning, 4514) by the acoustic liquid conveyor (Echo 655), and the plate was centrifuged at 1000 rpm for 1 minute; 5 μL of PARP enzyme solution was transferred into the 384-well reaction plate, and the plate was centrifuged at 1000 rpm for 1 minute, and incubated at 25°C for 10 min; 5 μL of substrate solution was transferred into the 384-well reaction plate, and the plate was centrifuged at 1000 rpm for 1 minute, and incubated at 25°C for 60 minutes; finally, the mP signal value of FP (ex/em: 485 nm/520 nm) was read using the fluorescence polarisation module of the BMG PHERAstar FSX. Subsequently, the IC50 value was determined by fitting the data to a four-parameter nonlinear regression curve using GraphPad Prism 8 software. The inhibition rate was calculated according to formula 1, where RLUsample represents the readout of the compound well, RLUmax represents the readout of the solvent control well, and RLUmin represents the readout of the control well without the PARP1/2 enzyme. The $IC_{50}$ value was determined by fitting the data to a four-parameter curve (log(inhibitor) vs. response -- Variable slope) using GraphPad Prism software.

$$\text{Inhibition\%} = (1-(\text{RLUsample}-\text{RLUmin})/(\text{RLUmax}-\text{RLUmin}))\times 100\% \quad \text{(formula 1)}$$

Table 1 PARP-1 enzyme activity

| Compound | $IC_{50}$ (nM) |
|---|---|
| 1 | 2.74 |
| 2 | 10.80 |
| 3 | 10.51 |
| 4 | 3.38 |
| 5 | 17.07 |
| 6 | 10.5 |
| 7 | 12.54 |

(continued)

| Compound | $IC_{50}$ (nM) |
|---|---|
| 8 | 5.42 |
| 9 | 3.25 |
| 10 | 4.55 |
| 11 | 5.63 |
| 12 | 3.14 |
| 13 | 6.39 |
| 18 | 7.94 |

Conclusion: the compounds of the present invention, such as the example compounds, have significant inhibitory effects on PARP-1 enzyme activity in vitro.

**2. DLD-1 cell activity assay**

DLD-1 (150 cells/well/100 μL) (ATCC, CCL-221) and DLD-1 BRCA2 KO (1500 cells/well/100 μL) (from ICE BIOSCIENCE INC.) were inoculated into a 96-well plate (Greiner, 655090) at appropriate cell densities and incubated overnight in a cell incubator at 37°C. The test compound was diluted in the corresponding cell culture medium, and the mixture was added to the well plate and incubated for 7 days. On the fourth day, the medium was replaced with a fresh medium containing the same concentrations of the compounds. The CellTiter-Glo reagent (CTG, Vazyme, DD1101-03) and the culture plate were taken out and returned to room temperature, and then 60 μL of CTG reagent was added to the cells. The plate was then shaken for 2 minutes to ensure complete cell lysis and placed at room temperature for 30 minutes. Luminescence signal values were read using a microplate reader (PHERAstar FSX). The wells containing only culture medium were used as negative controls, and the DMSO-containing wells were used as positive controls. The inhibitory activity percentage of the compounds was normalised using the readings from the negative and positive controls. Calculation formula of inhibition rate:

Inhibition rate (%) = (fluorescence value of positive control group - fluorescence value of experimental group)/(fluorescence value of positive control group - fluorescence value of negative control group) * 100% (formula 2)

Table 2 DLD-1 cell activity

| Compound | $IC_{50}$ (nM) | Max inh.% 10 μM |
|---|---|---|
| 1 | 3.64 | 93.99 |
| 2 | 4.67 | 93.07 |
| 3 | 4.67 | 91.59 |
| 4 | 3.10 | 91.67 |
| 8 | 16.80 | 86.72 |
| 9 | 7.99 | 81.75 |
| 10 | 6.96 | 87.64 |

Conclusion: the compounds of the present invention, such as the example compounds, exhibit good inhibitory activity.

**3. PARP selectivity experiment**

A549WT, A549 PARP1 KO and A549 PARP2 KO cell lines were inoculated into a 384-well plate at 2500/40 μL/well, and incubated overnight at 37°C with 5% $CO_2$. On the next day, 40 nL of serially diluted compounds were added, and the cells were incubated at 37°C with 5% $CO_2$ for 1 hour. The cells were stimulated by adding 5 μL of $H_2O_2$. A549 WT

and A549 PARP2 KO were stimulated with a final concentration of 200 μM for 5 minutes, and A549 PARP1 KO was stimulated with a final concentration of 20 mM for 45 minutes. After the stimulation, the supernatant was discarded, and the cells were fixed with 40 μL of pre-cooled methanol in a refrigerator at 4°C for 20 minutes. The cells were washed twice with PBS, and blocked with 20 μL of blocking buffer for 1 hour. A primary antibody mixture (prepared by diluting the pADPr antibody (10H: sc-56198) 1 : 500 in blocking buffer) was added, and the plate was incubated overnight at 4°C. The cells were washed three times with PBST. A secondary antibody mixture (prepared by diluting the goat anti-mouse IgG (H+L) highly cross-adsorbed secondary antibody (Alexa Fluor™ 488) 1 : 500 in blocking buffer) was added, and the plate was incubated at room temperature for 1 hour. The cells were washed three times with PBST, and 20 μL of DAPI solution (1 : 5000 dilution) was added. The plate was incubated at room temperature for 10 minutes before scanning.

Data analysis method

The stability of the assay was checked with DMSO and Talazoparib data:

H = Ave (DMSO); L = Ave (Talazoparib 5 μM); SD (H) = STDEV (DMSO); SD (L) = STDEV (Talazoparib 5 μM); CV% (H) = 100 * (SD_H/Ave_H); CV% (L) = 100* (SD_L/Ave_L); Z' = 1-3*(SD_H+ SD_L)/ (Ave_H - Ave_L); Inhibition% = (Ave_H-Sample) /(Ave_H-Ave_L)*100

The $IC_{50}$ of the compound was determined by fitting the data to a non-linear regression equation: Y = Bottom+(Top-Bottom)/(1+10^((LogIC50-X)*Hil Slope))

X: concentration of compound; Y: inhibition rate%; Top and Bottom: upper and lower plateaus in the unit of Y-axis. Hill slope: steepness of the curve.

Table 3 A549 PARP selectivity

| **Compound** | A549 PARP2 KO $IC_{50}$ (nM) | A549 PARP1 KO $IC_{50}$ (nM) |
|---|---|---|
| 2 | 0.63 | >5000 |

Conclusion: the compounds of the present invention, particularly the example compounds such as Compound 2, exhibit good selectivity for PARP1.

**4. Pharmacokinetic test in rats**

4.1. Experimental animals: Male SD rats (about 220 g, 6-8 weeks old, 6 rats/compound) purchased from CHENGDU DOSSY EXPERIMENTAL ANIMALS CO., LTD.

4.2 Experimental design: on the day of the experiment, 12 SD rats were randomly grouped according to their body weights. The animals were fasted with water available for 12-14 h one day before the administration, and were fed 4 h after the administration.

Table 4. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound 2 | 2.5 | 0.5 | 5 | Plasma | Intravenous administration |
| G2 | 3 | | 10 | 1 | 10 | Plasma | Intragastric administration |
| G3 | 3 | Compound 8 | 2.5 | 0.5 | 5 | Plasma | Intravenous administration |
| G4 | 3 | | 10 | 1 | 10 | Plasma | Intragastric administration |

Before and after administration, 0.15 ml of blood was taken from the orbit under isoflurane anaesthesia and placed in an EDTAK2 centrifuge tube. The tube was centrifuged at 5000 rpm at 4°C for 10 min, and plasma was collected. The blood collection time points for the intravenous administration group and intragastric administration group were: 0, 5 min, 15 min, 30 min, 1 h, 2 h, 4 h, 6 h, 8 h and 24 h. The brain tissues were collected 24 hours after the administration, rinsed with cold physiological saline to remove the residual blood on the surface, drained out and then homogenised. Before analysis and detection, all samples were stored at -80°C. The samples were quantitatively analysed by LC-MS/MS.

Table 5. Pharmacokinetic parameters of test compounds in plasma of rats

| Test compound | Administration mode | CL (mL/min/kg) | $Vd_{ss}$ (L/kg) | $AUC_{0-t}$ (hr*ng/mL) | F (%) | Brain/Plasma Ratio (24 h) |
|---|---|---|---|---|---|---|
| Compound 2 | i.v. (2.5 mg/kg) | 2.22±0.11 | 0.348±0.027 | 18739±972 | - | 1.03 |
| | i.g. (10 mg/kg) | - | - | 50042±11526 | 66.8±15 | |
| Compound 8 | i.v. (2.5 mg/kg) | 4.36±0.74 | 0.855±0.16 | 5722±298 | - | 0.242 |
| | i.g. (10 mg/kg) | - | - | 31816±8480 | 81.9±22 | |

Conclusion: the compounds of the present invention, such as example compounds 2 and 8, have good pharmacokinetic characteristics in rats.

**5. Pharmacokinetic test in mice**

5.1. Experimental animals: male Balb/c mice, 20-25 g, 12 mice/compound, purchased from CHENGDU DOSSY EXPERIMENTAL ANIMALS CO., LTD.

5.2 Experimental design: On the day of the experiment, the Balb/c mice were randomly grouped according to their body weights. The animals were fasted with water available for 12-14 h one day before the administration, and were fed 4 h after the administration.

Before and after administration, 0.06 mL of blood was taken from the orbit under isoflurane anaesthesia and placed in an EDTAK2 centrifuge tube. The tube was centrifuged at 5000 rpm at 4°C for 10 min, and plasma was collected. The blood collection time points for the intravenous group and intragastric administration group were: 0 min, 5 min, 15 min, 30 min, 1 h, 2 h, 4 h, 6 h, 8 h, and 24 h. The brain tissues were collected at 30 min, 2 h and 24 h after administration of compounds. The brain tissues were collected 24 hours after compounds, rinsed with cold physiological saline to remove the residual blood on the surface, drained out and then homogenised. Before analysis and detection, all samples were stored at -80°C. The samples were quantitatively analysed by LC-MS/MS.

Conclusion: the compounds of the present invention, such as the example compounds, have good pharmacokinetic characteristics in mice.

**6. Pharmacokinetic test in beagle dogs**

**6.1. Experimental animals:** male beagle dogs, about 8-11 kg, 6 beagle dogs/compound, purchased from BEIJING MARSHALL BIOTECHNOLOGY CO., LTD.

**6.2 Experimental method:** On the day of the experiment, the beagle dogs were randomly grouped according to their body weights. The animals were fasted with water available for 12-14 h one day before the administration, and were fed 4 h after the administration.

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for the intravenous administration group and intragastric administration group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were analysed quantitatively by LC-MS/MS.

**Conclusion:** the compounds of the present invention, such as the example compounds, have good pharmacokinetic characteristics in dogs.

**7. Pharmacokinetic test in monkeys**

**7.1. Experimental animals:** male cynomolgus monkeys, 3-5 kg, 3-6 years old, 4 monkeys/compound, purchased from SUZHOU XISHAN BIOTECHNOLOGY CO., LTD.

**7.2 Experimental method:** On the day of the experiment, the monkeys were randomly grouped according to

their body weights. The cynomolgus monkeys were fasted with water available for 14-18 h one day before the administration, and were fed 4 h after the administration.

Before and after the administration, 1.0 mL of blood was taken from the limb veins and placed in an EDTAK2 centrifuge tube. Centrifugation was performed at 5000 rpm at 4°C for 10 min, and the plasma was collected. The blood collection time points for the intravenous administration group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were quantitatively analysed by LC-MS/MS.

Conclusion: the compounds of the present invention, such as the example compounds, have good pharmacokinetic characteristics in monkeys.

## Claims

**1.** A compound as represented by formula I-1 or formula I, or a stereoisomer or pharmaceutically acceptable salt thereof,

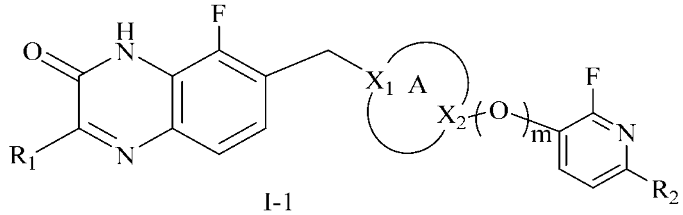

I-1

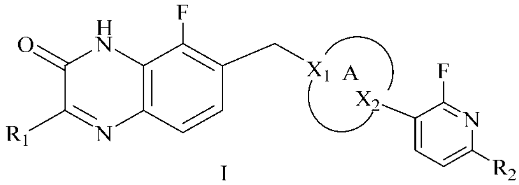

I

**characterised in that**

ring A is selected from

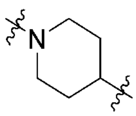,
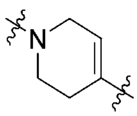,
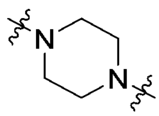 or
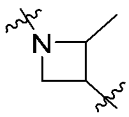;

$R_1$ is $C_{1-4}$ alkyl or $C_{3-6}$ cycloalkyl, wherein the alkyl or cycloalkyl is optionally substituted with 1-3 groups selected from D, halogen, OH, CN or $NH_2$;
$R_2$ is $-CONHR_A$ or $-NHCOR_A$;
each $R_A$ is independently $C_{3-6}$ cycloalkyl, or 5- to 6-membered heteroaryl substituted with 1-3 halogen, wherein the heteroaryl contains 1-3 heteroatoms selected from N, S or O, and the heteroaryl or cycloalkyl is optionally substituted with 1-3 groups selected from D, =O, $C_{1-4}$ alkyl, $C_{1-4}$ alkoxy, $-NHC_{1-4}$ alkyl,$-CONHC_{1-4}$ alkyl, $-NHCOC_{1-4}$ alkyl, OH, CN, $NH_2$ and $C_{1-4}$ haloalkyl; the cycloalkyl is substituted with at least one $=CH_2$, $=CF_2$, $=CHF$, $=CH(C_{1-6}$ alkyl) or $=C(C_{1-6}$ alkyl$)_2$;
m is 0 or 1;
the compound is not

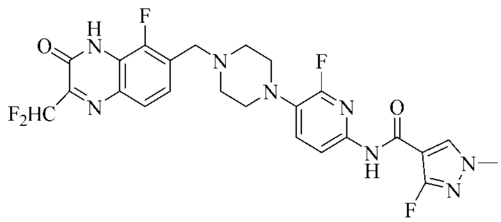.

2. The compound, or the stereoisomer or pharmaceutically acceptable salt thereof according to claim 1, **characterised in that**

ring A is selected from

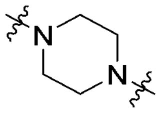 or 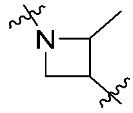;

$R_1$ is $C_{1-2}$ alkyl or $C_{3-4}$ cycloalkyl, wherein the alkyl or cycloalkyl is optionally substituted with 1-3 groups selected from D, F, Cl, OH, CN or $NH_2$; preferably, $R_1$ is methyl, ethyl, isopropyl, cyclopropyl or cyclobutyl, wherein the methyl, ethyl, isopropyl, cyclopropyl or cyclobutyl is optionally substituted with 1, 2 or 3 groups selected from D, F, Cl, OH, CN or $NH_2$;

each $R_A$ is independently $C_{3-6}$ cycloalkyl, or 5- to 6-membered heteroaryl substituted with 1-3 halogen, wherein the heteroaryl contains 1-3 heteroatoms selected from N, S or O, and the heteroaryl is optionally substituted with 1-3 groups selected from D, =O, $C_{1-4}$ alkyl, OH, CN, $NH_2$ or $C_{1-2}$ haloalkyl; the cycloalkyl is substituted with one $=CH_2$, $=CF_2$, =CHF, $=CH(C_{1-2}$ alkyl) or $=C(C_{1-2}$ alkyl$)_2$; preferably, each $R_A$ is independently 5-membered heteroaryl substituted with 1, 2 or 3 F or Cl,

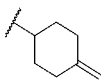, 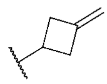, 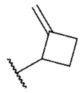,

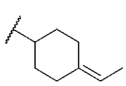, 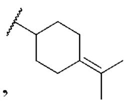, 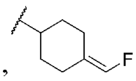, 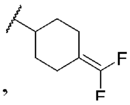,

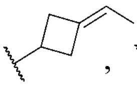, 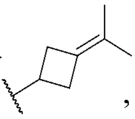, 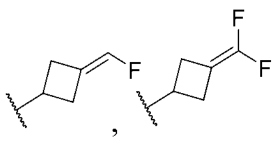, 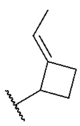, 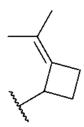, 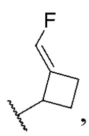, 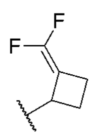,

wherein the heteroaryl contains 1, 2 or 3 heteroatoms selected from N, S or O, and the heteroaryl is optionally substituted with 1, 2 or 3 groups selected from D, =O,$-CH_3$, $-CH_2CH_3$, $-CH_2CH_2CH_3$, $-CH(CH_3)CH_3$, $-CH(CH_3)CH_2CH_3$, OH, CN or $NH_2$.

3. The compound, or the stereoisomer or pharmaceutically acceptable salt thereof according to claim 1 or 2, **characterised in that**

ring A is selected from

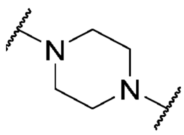 or 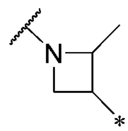,

wherein the "~" end is connected to the left side, and the " * " end is connected to the right side;
$R_1$ is selected from $-CH_3$, $-CH_2CH_3$ or $-CHF_2$;
each $R_A$ is independently selected from

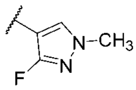, 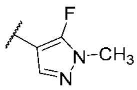, 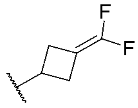,

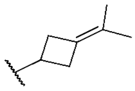 or 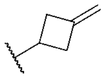.

**4.** The compound as represented by formula I, or the stereoisomer or pharmaceutically acceptable salt thereof according to claim 1 or 2, **characterised in that**

ring A is

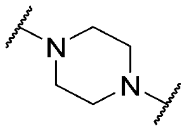,

wherein the "~" end is connected to the left side, and the " * " end is connected to the right side;
$R_1$ is selected from $-CH_3$, $-CH_2CH_3$ or $-CHF_2$;
each $R_A$ is independently selected from

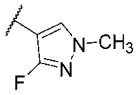, 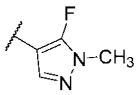, 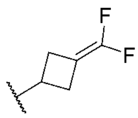,

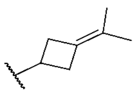 or 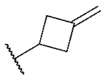.

**5.** The compound as represented by formula I-1, or the stereoisomer or pharmaceutically acceptable salt thereof according to claim 1 or 2, **characterised in that**

ring A is

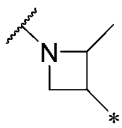,

wherein the "~" end is connected to the left side, and the " * " end is connected to the right side;
$R_1$ is selected from $-CH_3$, $-CH_2CH_3$ or $-CHF_2$; m is 1;
each $R_A$ is independently selected from

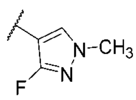, 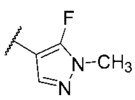, 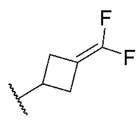,

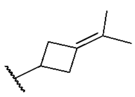 or 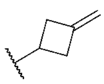.

6. A compound as represented by formula I-1 or formula I, or a stereoisomer or pharmaceutically acceptable salt thereof, **characterised in that** the compound has a structure selected from one of

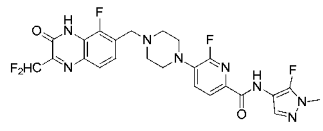
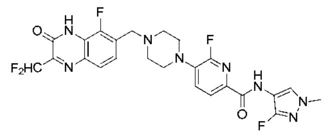
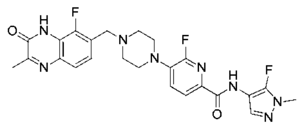
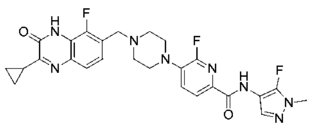
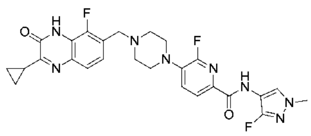
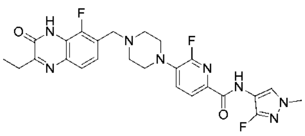
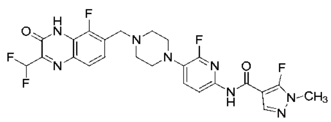
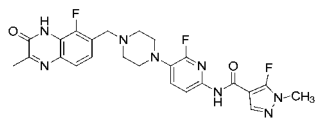
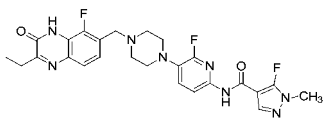
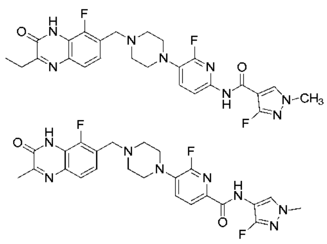
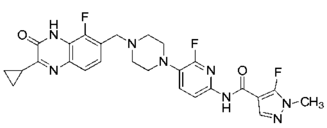
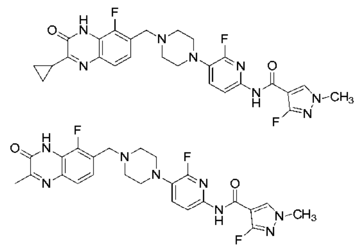
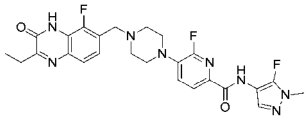
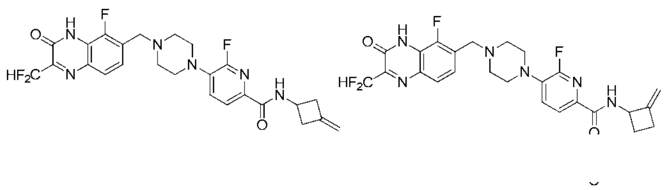
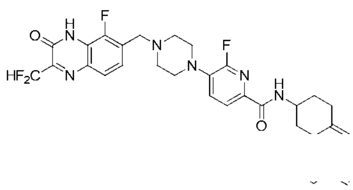
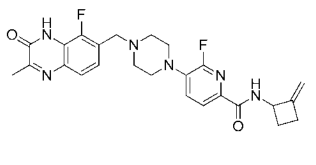
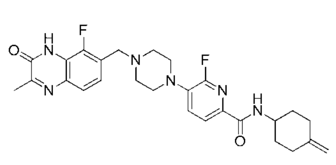
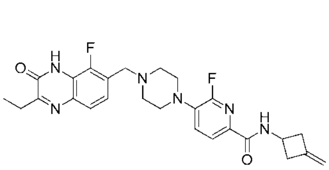
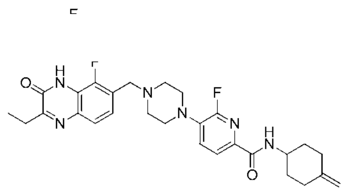
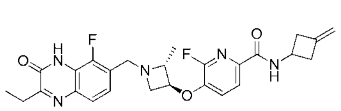
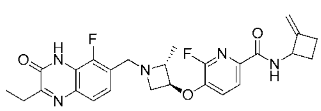
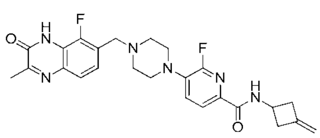
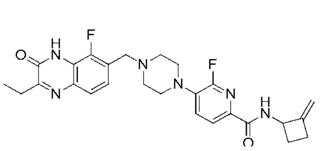
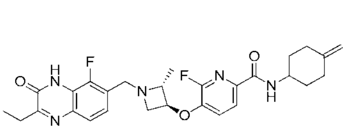

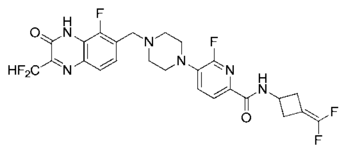
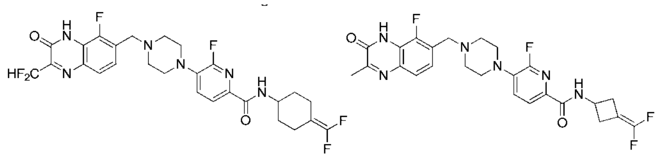
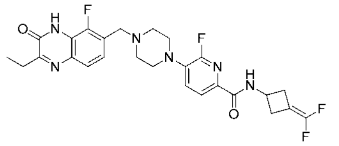
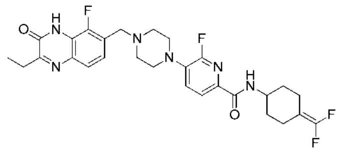
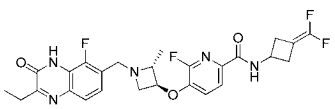
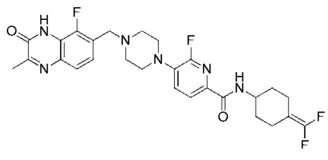
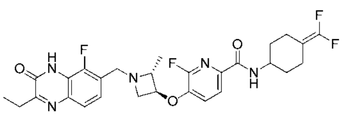
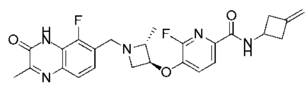
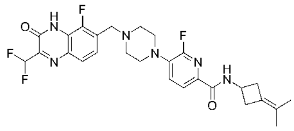
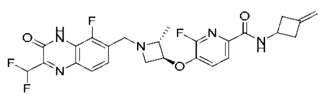
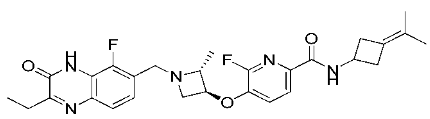.

7. A pharmaceutical composition, **characterised by** comprising the compound, or the stereoisomer or pharmaceutically acceptable salt thereof according to any one of claims 1-6, and a pharmaceutically acceptable carrier and/or excipient.

8. Use of the compound, or the stereoisomer or pharmaceutically acceptable salt thereof according to any one of claims 1-6, or the composition according to claim 7 in the preparation of a drug for treating/preventing a PARP1-mediated disease.

9. The use according to claim 8, **characterised in that** the PARP1-mediated disease is selected from breast cancer, uterine cancer, cervical cancer, ovarian cancer or prostate cancer.

10. A pharmaceutical composition or pharmaceutical preparation, **characterised in that** the pharmaceutical composition or pharmaceutical preparation comprises 1-1440 mg of the compound, or the stereoisomer or pharmaceutically acceptable salt thereof according to any one of claims 1-6, and a pharmaceutically acceptable carrier and/or excipient.

11. A method for treating a disease in a mammal, **characterised in that** the method comprises administering to a subject a therapeutically effective amount of the compound, or the stereoisomer or pharmaceutically acceptable salt thereof according to any one of claims 1-6, wherein the therapeutically effective amount is preferably 1-1440 mg, and the disease is preferably breast cancer, uterine cancer, cervical cancer, ovarian cancer or prostate cancer.

## INTERNATIONAL SEARCH REPORT

International application No.
**PCT/CN2024/098857**

**A. CLASSIFICATION OF SUBJECT MATTER**

C07D401/14(2006.01)i; C07D471/04(2006.01)i; C07D403/14(2006.01)i; A61K31/4353(2006.01)i; A61K31/495(2006.01)i; A61K31/498(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of science, Registry(STN), Caplus(STN), MARPAT(STN): 海思科, PARP, BRCA, 喹喔啉, 含氮杂环, 抑制剂, 聚合酶, haisco, quinoxaline, nitrogen-containing heterocyclic, inhibitor, polymerase, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2022228387 A1 (FOCHON BIOSCIENCES, LTD.) 03 November 2022 (2022-11-03) claims 1, 6, 8, 12 and 20-23, and description, embodiment 378 | 1-11 |
| X | WO 2023056039 A1 (XINTHERA, INC.) 06 April 2023 (2023-04-06) claims 1 and 81-85, and description, page 43 | 1-11 |
| PX | WO 2023227052 A1 (TIBET HAISCO PHARMACEUTICAL CO., LTD.) 30 November 2023 (2023-11-30) claims 1, 3, 4 and 8-13 | 1-11 |
| PX | CN 116891456 A (SHANGHAI HANSOH BIOMEDICAL CO., LTD. et al.) 17 October 2023 (2023-10-17) claims 1, 3 and 9-13 | 1-11 |
| A | WO 2023046034 A1 (MINGHUI PHARMACEUTICAL (HANGZHOU) LIMITED et al.) 30 March 2023 (2023-03-30) claims 1-16 | 1-11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 August 2024** | **26 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/098857**

**C. DOCUMENTS CONSIDERED TO BE RELEVANT**

| Category* | Citation of document, with indication, where appropriate, of the relevant passages | Relevant to claim No. |
|---|---|---|
| A | WO 2023066363 A1 (HAISCO PHARMACEUTICAL GROUP CO., LTD.) 27 April 2023 (2023-04-27) claims 1-12 | 1-11 |
| A | WO 2023046149 A1 (ZHANG, Wenyan) 30 March 2023 (2023-03-30) claims 1-11 | 1-11 |
| A | WO 2023046158 A1 (ZHANG, Wenyan) 30 March 2023 (2023-03-30) claims 1-14 | 1-11 |

Form PCT/ISA/210 (second sheet) (July 2022)

**INTERNATIONAL SEARCH REPORT**

International application No.
**PCT/CN2024/098857**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:

Claim 11 falls within disease treatment methods as defined in PCT Rule 39.1(iv). The present search report is provided on the basis of the use of a compound in the preparation of a drug for treating related diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/098857**

| Patent document cited in search report | Publication date (day/month/year) | Patent family member(s) | Publication date (day/month/year) |
|---|---|---|---|
| WO 2022228387 A1 | 03 November 2022 | TW 202309027 A | 01 March 2023 |
| | | CN 117321051 A | 29 December 2023 |
| WO 2023056039 A1 | 06 April 2023 | US 2023203033 A1 | 29 June 2023 |
| | | US 11802128 B2 | 31 October 2023 |
| | | DOP 2024000060 A | 15 May 2024 |
| | | CO 2024003949 A2 | 18 April 2024 |
| | | IL 311376 A | 01 May 2024 |
| | | TW 202322807 A | 16 June 2023 |
| | | KR 20240069791 A | 20 May 2024 |
| | | AU 2022356286 A1 | 16 May 2024 |
| | | US 2023159525 A1 | 25 May 2023 |
| | | CA 3232775 A1 | 06 April 2023 |
| WO 2023227052 A1 | 30 November 2023 | TW 202400171 A | 01 January 2024 |
| CN 116891456 A | 17 October 2023 | None | |
| WO 2023046034 A1 | 30 March 2023 | None | |
| WO 2023066363 A1 | 27 April 2023 | CA 3235698 A1 | 27 April 2023 |
| | | TW 202322811 A | 16 June 2023 |
| | | AU 2022373697 A1 | 30 May 2024 |
| | | CN 118139860 A | 04 June 2024 |
| WO 2023046149 A1 | 30 March 2023 | None | |
| WO 2023046158 A1 | 30 March 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021013735 A1
- US 2022009901 A1
- WO 2022212538 A1
- WO 2021260092 A
- WO 2020249664 A1
- WO 2023056039 A1
- US 20220009901 A1
- WO 2023025324 A1
- DD 110103